# EUROPÄISCHE PATENTSCHRIFT

(11) **EP 1 946 039 B1**
(45) Veröffentlichungstag und Bekanntmachung des Hinweises auf die Patenterteilung: **29.04.2009**
(21) Anmeldenummer: 06804859.4
(22) Anmeldetag: 08.11.2006
(51) Int. Cl.: G01B 9/02, A61B 3/10

(54) **VERFAHREN UND VORRICHTUNG ZUR ERMITTLUNG GEOMETRISCHER WERTE AN EINEM GEGENSTAND**
METHOD AND APPARATUS FOR DETERMINATION OF GEOMETRIC VALUES ON AN OBJECT
PROCEDE ET DISPOSITIF DE DETERMINATION DE GRANDEURS GEOMETRIQUES SUR UN OBJET

(30) Priorität: 10.11.2005 EP 05405625
(43) Veröffentlichungstag der Anmeldung: 23.07.2008
(73) Patentinhaber: HAAG-STREIT AG, 3098 Köniz (CH)
(72) Erfinder: WÄLTI, Rudolf, CH-3097 Liebefeld (CH); BURI, Urs, CH-3205 Mauss (CH); BREITENSTEIN, Jörg, CH-3052 Zollikofen (CH)
(74) Vertreter: Rüfenacht, Philipp Michael
(86) Internationale Anmeldenummer: PCT/CH2006/000625
(87) Internationale Veröffentlichungsnummer: WO 2007/053971

(56) Entgegenhaltungen:
- WO-A-01/38820
- WO-A-03/086180
- DE-A1- 3 446 014
- US-A- 5 301 010
- US-B1- 6 198 540

## Beschreibung

### Technisches Gebiet

Die Erfindung betrifft ein Verfahren und eine Vorrichtung zur Ermittlung geometrischer Werte wenigstens zweier voneinander distanzierter Bereiche bei einem transparenten oder diffusiven Gegenstand, insbesondere zur Ermittlung von Schichtdicken und Längen und / oder Flächenkrümmungen (Topographie) als geometrische Werte. Unter geometrischen Werten werden beispielsweise Schichtdicken, Abstände, Längen und Topographien verstanden.

### Stand der Technik

In der Regel arbeiten optische Zeitraum-Kohärenztomographie-Systeme (time domain optical coherence tomography "TDOCT") mit einem Verschieben eines Zeitfensters. Bei einem derartigen konventionellen System wird die Länge eines Referenzarmes eines Michelson-Interferometers entsprechend einer an einem Messobjekt auszumessenden Länge bewegt. Eine Interferenzerscheinung tritt im Detektorarm des Michelson-Interferometers immer dann auf, wenn die Laufzeit einer Strahlung im Messarm gleich einer Strahlungslaufzeit im Referenzarm ist. Um diese Interferenzerscheinung bei den bekannten Vorrichtungen zu detektieren, wird ein einziger Detektor benötigt.

Eine Alternative zu dem in der Länge veränderbaren Referenzarm des TDOCTs ist eine Messung einer spektralen Dichte in einem Michelson-Interferometer unter Verwendung eines Spektometers; man spricht dann von optischer Spektralraum-Kohärenztomographie (spectral domain optical coherence tomography "SDOCT"). Hierbei muss keine Längenveränderung des Referenzarmes vorgenommen werden. Es wird lediglich eine Einheit verwendet, welche eine Phasenverschiebung verursacht. Die vom Spektrometer in Spektralbereich aufgeteilte Strahlung wird dann auf eine Reihe von Detektoren geführt und die so erhaltenen elektrischen Signale über eine Fourier Transformation ausgewertet

Unter Verwendung von TDOCT liessen sich optische Dicken mit Michelson-Interferometem schnell und genau ermitteln. Bekannte Michelson-Interferometer hatten einen Mess- und einen Referenzarm. Die optische Armlänge des Referenzarms war entsprechend den Messlängen im Messarm beispielsweise durch Verschieben eines Retroreflektors veränderbar. Bekannte Michelson-Interferometer hatten ferner eine Strahlungsquelle, deren Strahlung in den Mess- und in den Referenzarm aufgeteilt wurde. Im Messarm wurde der Gegenstand (Messobjekt) angeordnet, bei dem geometrische Werte, wie beispielsweise eine Schichtdicke, eine Länge oder eine Topografie bestimmt werden sollten. Die vom Gegenstand und von einem Spiegel im Referenzarm reflektierten Strahlen wurden überlagert. Eine Interferenz der von der Schichtvorder- und von der Schichtrückseite reflektierten Strahlen trat immer dann ein, wenn die optischen Laufzeiten der Strahlungen im Mess- und im Referenzarm gleich lang waren. Es wurde vorzugsweise eine Strahlungsquelle mit kurzer Kohärenzlänge verwendet. Der Verschiebeweg des Spiegels oder eine sonstige definierte optische Laufzeitveränderung im Referenzarm gab dann unter Berücksichtigung des Brechungsindexes der Schicht die jeweilige Schichtdicke an.

Sollten exakte Schichtdicken von annähernd bekannten Schichtdicken ermittelt werden, wie z. B. beim menschlichen Auge, so wurde zur Verringerung der Messzeit und zum Ausschliessen von Fehlmessungen durch nicht definierte Reflexionsorte, der Referenzarm in zwei Teilreferenzarme aufgeteilt. Eine erste optische Referenzarmlänge definierte dann eine optische Laufzeit bis zur Schichtoberfläche und eine zweite optische Referenzarmlänge die optische Laufzeit bis zur "standardisierten" Schichtunterseite. Lediglich die Abweichungen vom "Standard" wurden somit ermittelt. Diese zwei Teilreferenzarme zeigte z. B. die Figur 12 in der US 5,301,010. Es konnte allerdings auch ein einziger Referenzarm verwendet werden, der zwei in Strahlrichtung versetzte, als Stufenspiegel ausgebildete Spiegel hatte; eine derartige Anordnung zeigte die US 5,301,010 z. B. in Figur 9.

Mit gleichem Ergebnis konnte jedoch auch eine Strahlteilumlenkung im Messstrahl, wie in der WO 01/38820 beschrieben, vorgenommen werden. In der WO 01/38830 wurde ein direkter erster Messstrahl etwa auf die Schichthinterseite fokussiert. Ein aus dem ersten Messstrahl teilweise ausgeblendeter zweiter Messstrahl wurde über einen Umweg umgelenkt und im Umweg derart fokussiert, dass sein Fokuspunkt etwa auf der Schichtvorderseite zu liegen kam. Die Umweglänge entsprach dann der zu erwartenden Standardschichtdicke. Es war jetzt lediglich ein einziger Referenzarm mit einem Wegvariationselement vorhanden.

Aus der US 6,198,540 (Kowa) ist ein Kohärenztomograph mit mehreren Referenzarmen bekannt. Ein Strahlaufteiler erzeugt für die Referenzarme mehrere verschiedene optische Pfade, in welchen die Weglänge mit geeigneten Mitteln variiert werden kann. Eine der Ausführungsformen zeigt ein Paket von parallel nebeneinander angeordneten Scheiben mit jeweils spiralförmig verlaufender, reflektierender Mantelfläche und von Scheibe zu Scheibe unterschiedlichem Durchmesser. Durch die Rotation des Scheibenpaketes verändert sich die Weglänge während einer Umdrehung kontinuierlich. Die parallel zueinander auf die unterschiedlichen Spiralscheiben gerichteten Referenzstrahlen erlauben es, gleichzeitig unterschiedliche Grundlaufzeiten zu realisieren, wobei die Grundlaufzeiten durch die rotierenden Spiralscheiben kontinuierlich variiert werden während eines Umlaufs.

Aus der WO 03/086180 (Haag-Streit) ist eine Messanordnung als Teil des ophthalmologischen Untersuchungs- und/oder Behandlungsplatzes bekannt, welche eine Michelson-Interferometer mit kurzkohärenter Strahlungsquelle aufweist. Im Messarm ist ein optisch transparenter und/oder diffusiver, reflektierender Gegenstand einbringbar und im Referenzarm **(5)** ein rotierender Würfel als Weglängenvariationseinheit zur Laufzeitänderung. Weiter sind wenigstens zwei einen Laufzeitunterschied hervorrufende Reflektoren vorhanden. Die Messanordnung dient zur Messung optischer Eigenschaften wenigstens zweier voneinander distanzierter Bereiche beim transparenten und/oder diffusiven Gegenstand mit einer Messzeit im Subsekundenbereich.

Bei den oben skizzierten Anordnungen zur Schichtdickenbestimmung handelte es sich um aufwendige optische Anordnungen.

### Darstellung der Erfindung

### Aufgabe der Erfindung

Aufgabe der Erfindung ist es, ein Verfahren sowie eine Vorrichtung vorzustellen, bei denen in einer kompakten, verhältnismässig einfachen Anordnung mit geringem Justageaufwand zur Ermittlung von geometrischen Werten an transparenten bzw. diffusiven Gegenständen gearbeitet werden kann.

### Lösung der Aufgabe

Die Aufgabe wird durch die Merkmale des Anspruchs 1 bzw. des Anspruchs 6 gelöst. Ein an sich bekanntes Michelson-Interferometer wird als optischer Zeitraum-Kohärenztomograph mit wenigstens zwei Referenzarmen unterschiedlicher optischer Länge zur Ermittlung von Schichtdicken verwendet, wobei ein Unterschied der optischen Weglänge in den wenigstens zwei Referenzarmen einer "Standard"-Schichtdicke an bzw. in einem transparenten oder diffusiven Gegenstandes entspricht. Unter einer Standard-Schichtdicke wird eine Schichtdicke verstanden, deren generelle Schichtdicke zwar bekannt ist, es aber auf Abweichungen zu dieser generell bekannten Schichtdicke ankommt, welche ermittelt werden sollen. Die von der Strahlungsquelle des Michelson-Interferometers ausgehende Strahlung hat im Vergleich zu den auszumessenden Schichtdicken eine kurze Kohärenzlänge.

Generell bekannte Schichtdicken sind z. B. beim menschlichen Auge die Dicke der Cornea, die Tiefe der Vorderkammer, die Linsendicke und die Glaskörpertiefe oder Kombinationen davon; es können selbstverständlich auch Längen, wie die Augenlänge ermittelt werden. Neben Schichtdicken in organischen Gegenständen, können auch Schichtdicken aus anorganischem Material bestimmt werden, wie beispielsweise Foliendicken, Beschichtungsdicken usw. Das an sich bekannte Michelson- interferometer hat nun ein rotierendes Wegvariationselement, wie es beispielsweise in der EP 0 877 913 und der DE 34 46 014 beschrieben ist.

Im Gegensatz zu den bekannten Michelson-Interferometern mit wenigstens zwei Referenzarmen ist nun dieses eine rotierende Weglängenvariationselement wirksames Teil in den wenigstens beiden Referenzarmen, wobei die durch das Weglängenvariationselement erzeugten Weglängenänderungen vorzugsweise über gleichlange Bereiche laufen. Die Strahlen der beiden Referenzarme werden nun nicht parallel sondern derart in einem Versetzungswinkel (d.h. schräg) zueinander auf dieses eine Weglängenvariationselement geführt, dass die Weglängenvariation zuerst in dem einen Referenzarm und dann in dem anderen Referenzarm wirkt. Durch diese aufeinander folgende Wirkung sind die der Schichtvorder- und der Schichthinterseite (bzw. einem Abstandsanfang und einem Abstandsende) entsprechenden Interferenzen einwandfrei voneinander zu trennen, da sie immer zeitlich aufeinander folgend auftreten. Die Ausrichtung der wenigstens beiden Referenzarme und des rotierenden Weglängenvariationselements können nun noch derart vorgenommen werden, dass zur Trennung der beiden Signale aus den wenigstens beiden Referenzarmen ein Unterbruch entsteht, in welchem keine Strahlungsreflexion in den Referenzarmen über einen vorgegebenen Zeitraum eintrifft.

Vorzugsweise ist die Rotationsachse des Wegvariationselements parallel zu den planen Mantelflächen angeordnet, wobei der bzw. jeder Referenzstrahl senkrecht zur Rotationsachse (d.h. in einer senkrecht zur Rotationsachse stehenden geometrischen Ebene) auf die betreffende Mantelfläche trifft. Es muss nicht jeder Referenzstrahl derartig geführt werden; es sind auch schräge (d.h. von 90° verschiedene) Auftreffwinkel möglich, wodurch die Weglängenvariationslänge verlängerbar wäre. Die Ermittlung der zu erreichenden Weglängenvariationen sowie deren möglichst linear über dem Drehwinkel verlaufenden Weglängenänderung ist jedoch bei zur Achse senkrechter Anordnung am einfachsten zu konstruieren und zu berechnen.

Die Strahlungen im Mess- sowie in den Referenzarmen werden möglichst immer in Strahlungsleitern geführt. Lediglich vor und hinter dem Wegvariationselement sowie dem Messobjekt und anderen optischen Komponenten muss eine Aus- bzw. Einkopplung in Strahlungsleiter erfolgen. Die Verwendung von Strahlungsleitem auf möglichst dem gesamten Weg im Messarm und den Wegen in den Referenzarmen gestattet einen robusten und störungsfreien Aufbau der Vorrichtung. In der untenstehenden Beschreibung wird somit immer von einem Strahl gesprochen, wenn es sich um nicht in einem Strahlungsleiter geführte, und somit freie Raumstrahlung handelt, welche eben eine Strahlkonfiguration aufweist; in den Strahlungsleitem wird von Strahlung gesprochen.

Um ausgeprägte Interferenzsignale auch bei nur schwach reflektierenden Ober- bzw. Unterseiten der Schichten im Messarm zu erhalten, wird man die Messstrahlung im Messarm auf die Schichtober- bzw. -unterseite fokussieren. Die Messmethode ist sehr empfindlich; es sollten wenigstens 10⁻⁴% der jeweils auf die Schichtober- oder -unterseite fallenden Strahlungsintensität reflektiert werden. Diese Fokussierung muss jedoch synchronisiert bzw. periodisch mit der Rotation des Weglängenvariationselements erfolgen. D. h. immer dann, wenn beispielsweise der eine Referenzarm zusammen mit dem Weglängenvariationselement die Schichtoberseite ausmisst, muss auch der Messstrahl auf die Schichtoberseite fokussiert werden, und wenn dann anschliessend der andere Referenzarm die Schichtunterseite ausmisst, muss der Messstrahl auf die Schichtunterseite fokussiert sein.

Es sei hierzu bemerkt, dass das in der EP 0 877 913 im Detail beschriebene Wegvariationselement einen quadratischen Querschnitt hat und beispielsweise mit einer Rotationsfrequenz von 2,5 Hz betrieben wird, wobei dann, wie unten beschrieben ist, sich eine Umschaltfrequenz für die Fokuspunkte bei lediglich zwei Referenzarmen von 20 Hz ergibt.

Anstelle eines quadratischen Querschnitts, in dem die Referenzstrahlung verläuft, können selbstverständlich auch andere regelmässige Vielecke als Querschnitt verwendet werden. Neben regelmässigen Vielecken als Querschnitt können auch analog zur DE 34 46 014 sternförmige Querschnitte mit Aussenverspiegelung verwendet werden.

Den Querschnitt wird man in der Regel als regelmässiges Vieleck ausbilden, muss es aber nicht. Es können auch unregelmässige Vielecke verwendet werden. In diesem Fall ist jedoch ein dem Grundweg des Referenzarms überlagerter Variationsweg je nach Strahlungseintritt durch eine bestimmte Vieleckfläche unterschiedlich lang, was nicht unbedingt hinderlich ist, da die jeweilige Variationsweglänge abgespeichert mit dem jeweiligen Drehwinkel verknüpft ist. Analog kann auch ein unregelmässiger Stern der DE 34 46 014 verwendet werden.

Eine Fokuspunktverschiebung kann nun durch eine entsprechende Verschiebung einer Fokussierlinse erfolgen. Ein Verschieben einer Linse hat nun den Nachteil, dass die Linse genau auf der Strahlachse verschoben werden muss, ansonsten ergibt sich eine laterale Verschiebung (Verkippung) zwischen den Messpunkten auf der Schichtober- und der -unterseite; es würden "schräge" Schichtdicken gemessen.

Es wird hier nun vorgeschlagen, nicht die Fokussierlinse zu verschieben, sondern in den nicht parallelen Strahlengang vor oder hinter einer entsprechenden Fokussierlinse ein transparentes Material mit vorgegebener Dicke und mit vorgegebenem Brechungsindex einzubringen. Ist das Material im Strahlengang eingebracht, ergibt sich ein verschobener Fokusort gegenüber aus dem Strahlengang entferntem Material. Durch den Strahlengang könnte nun ein transparenter "Kamm" mit planparallelen Kammzinken mit entsprechender Geschwindigkeit geschoben werden, wobei die Kammzinken dann eine Fokuspunktlage und die Zwischenräume eine andere verschobene Fokuspunktlage definieren würden. Sind drei und mehr unterschiedliche Fokuspunkte notwendig könnten die Kammzinken gestuft auch mit unterschiedlichen Dicken und jeweils planparallelen Vorder- und Rückseiten ausgebildet werden.

Die Verwendung einer linearen Verschiebung hat jedoch den Nachteil, dass der Kamm, nach dem er einmal durchgelaufen ist, in die entgegen gesetzte Richtung mit einer entsprechenden Beschleunigung im Umkehrpunkt bewegt werden muss, was unterschiedliche Zinkenbreiten und -abstände bewirken würde. Man wird deshalb in vorteilhafter Wiese für eine schnelle Fokuspunktverschiebung eine rotierende Scheibe mit einer Rechteckzahnung verwenden, wobei die Zahnbereiche transparent mit entsprechendem Brechungsindex und mit entsprechender Dicke ausgewählt sind.

Die oben geschilderte Dickenbestimmung lässt sich, wie beschrieben, durchführen. Oftmals sind die auszumessenden Schichten jedoch nicht plan. Ein typisches Beispiel hierfür ist die Cornea des menschlichen Auges. Für eine Augenbehandlung ist es nun von besonderem Wert neben der Augenlänge, der Dicke der Cornea bzw. der Augenlinse von letzteren gleichzeitig die Oberflächenkrümmung zu ermitteln.

Um neben der oben geschilderten einfachen linearen Messungen (z. B. Dickenmessung) gleichzeitig auch noch die Topografie als weiteren geometrischen Wert bestimmen zu können, wird vor dem auszumessenden Gegenstand, hier dem Auge, eine optische Struktur, hervorgerufen durch unterschiedliche Leuchtdichten, verwendet. Die Strahlung dieser Struktur wird von der Augenoberfläche reflektiert, dann abgebildet und die Abbildung ausgewertet. Da die vor dem Gegenstand angeordnete, in der Regel flächige Struktur bekannt ist, kann aus der Abbildung auf die Krümmung geschlossen werden.

Die Ermittlung der Topografie wie auch die Ermittlung von Schichtdicken bzw. Längen erfolgt optisch. Es ist nun darauf zu achten, dass die für die optischen Verfahren verwendeten Strahlungen sich nicht gegenseitig stören. Man könnte nun für die beiden Ermittlungsverfahren den Strahlungen unterschiedliche Modulationsfrequenzen aufmodulieren oder mit unterschiedlicher Polarisation arbeiten. Vorzugsweise wird jedoch mit unterschiedlichen Strahlungswellenlängen arbeiten. Um Strahlen unterschiedlicher Wellenlängen überlagern zu können, wird mit einem wellenlängenselektiven Spiegel gearbeitet, der die eine Strahlung reflektierend umlenkt und die andere Strahlung möglichst ohne Verluste transmittiert. Vorzugsweise wird man den Messstrahl des Michelson-Interferometers reflektierend umlenken und die Strahlung zur Ermittlung der Topographie transmittieren lassen. Selbstverständlich könnte auch umgekehrt verfahren werden.

Die vor der auszumessenden Oberfläche anzuordnende, optische Struktur kann eine seit langem bekannte Placido-Scheibe (Placido disc) sein. Vorzugsweise wird man jedoch in einer vorgegebenen Struktur angeordnete Strahlungsquellen verwenden. Wie aus der räumlichen Struktur der Lichtquellen dann auf die Oberflächenkrümmung zurück geschlossen werden kann, ist beispielsweise in der DE 43 25 494, der US 5,325,134, der DD 251 497, der US 5,684,562, in der Veröffentlichung von M. R. Morelande et al., "Automatic Estimation of the Comeal Limbus in Videokeratooscopy", IEEE Trans. on Biomedical Eng., Vol. 49, No. 12, Dec.2002, S. 1617-1625 bzw. S.A. Klein, " Corneal Topography Reconstruction Algorithm that Avoids the Skew Ray Ambiguity and the Skew Ray Error", Optometry and Vision Sci., Vol. 74, No.11, Nov. 1997, S. 945-962 beschrieben.

Vorzugsweise wird man als Strahlungsquellen LEDs verwenden. Um bei der Auswertung einen Schwerpunkt des Strahlungs(Licht-)fleckes gut bestimmen zu können, kann den LEDs eine Streuscheibe im Strahlengang vorgeschaltet werden. Man kann zudem LEDs mit unterschiedlichen Wellenlängen verwenden, um z. B. bei stark deformierten Hornhäuten, bei deren Messung sich mehrere LED-Abbildungen überlappen könnten, eine eindeutige Zuordnung der LEDs zu ihren Bildern erhalten. Die Verwendung einer Streuscheibe hat den weiteren Vorteil, dass LEDs mit einer grösseren Strahlungs-(Leucht)-dichte als ohne Streuscheibe verwendet werden können.

Anstatt das Michelson-Interferometer als optischen Zeitraum-Kohärenztomographen (TDOCT) zu betreiben, kann es auch umgebaut als optischer Spektralraum-Kohärenztomograph (SDOCT) betrieben werden. Ein SDOCT ist ein sehr schnelles Messinstrument, es eignet sich somit insbesondere zur Verarbeitung von vielen Messpunkten, welche z. B. bei der Bestimmung einer Topographie oder einer dreidimensionalen Tomographie anfallen. Anstelle des oben skizzierten Einsatzes von mehreren LED-Strahlungsquellen, kann somit, wie unten ausgeführt ist, eine Topographie auch mit SDOCT ermittelt werden.

Weitere Ausführungsvariationen zur Erfindung und deren Vorteile ergeben sich aus dem nachfolgenden Text.

### Kurze Beschreibung der Zeichnungen

Nachfolgend werden Beispiele der erfindungsgemässen Vorrichtung und des erfindungsgemässen Verfahrens anhand von Zeichnungen näher erläutert. Es zeigen:
- Fig. 1: eine schematische Darstellung einer erfindungsgemässen Vorrichtung zur Ermittlung von Dicken bzw. Weglängen in optisch transparenten bzw. diffusiven Materialien,
- Fig. 2a-c: ein um eine Rotationsachse rotierbares Weglängenvariationselement der in Figur 1 dargestellten Vorrichtung,
- Fig. 3: eine skizzenhafte Darstellung eines Messkopfes der in Figur 1 dargestellten Vorrichtung,
- Fig. 4: eine Fokuspunktverschiebungseinheit als rotierende Scheibe zur Verstellung des Fokuspunktes eines Messstrahls der in Figur 1 dargestellten Vorrichtung,
- Fig. 5a-d: eine Ausführungsvariante der in Figur 4 dargestellten Fokuspunktverschiebungseinheit, welche aus zwei rotierenden Scheiben zusammengesetzt ist, wobei,

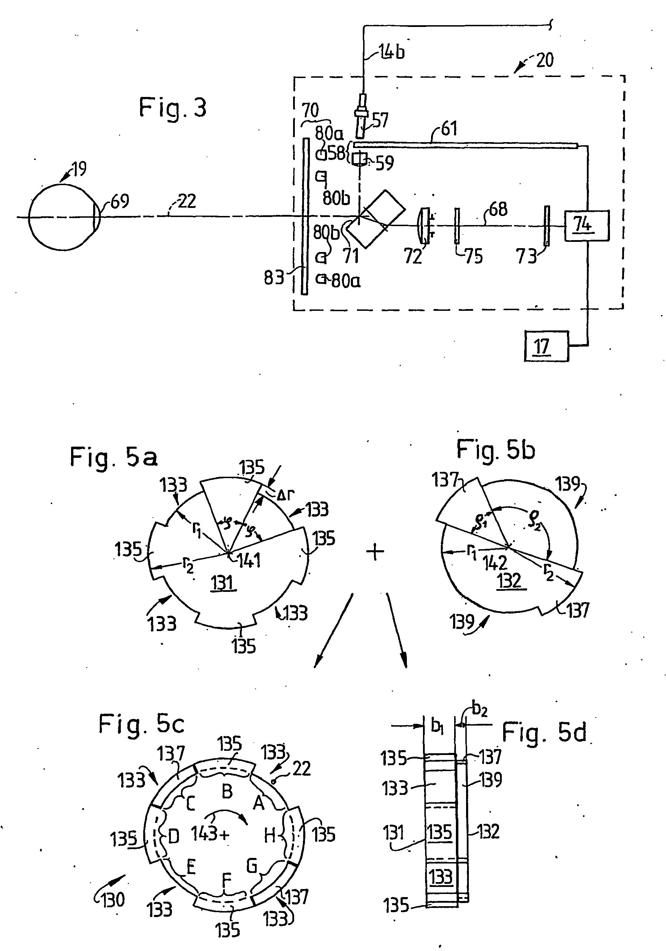

- Fig. 5a: eine Draufsicht auf die eine Scheibe,
- Fig. 5b: eine Draufsicht auf die andere Scheibe,
- Fig. 5c: eine Draufsicht auf die aus zwei Schieben bestehende Fokuspunktverschiebeeinheit und
- Fig. 5d: eine Seitenansicht der in Figur 5c dargestellten Einheit zeigen,

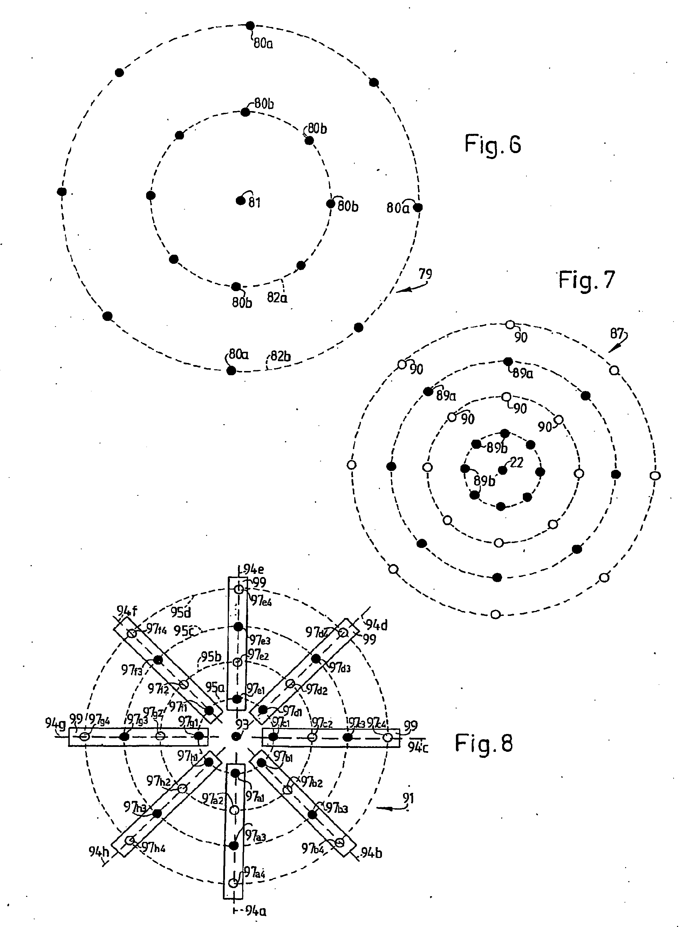

- Fig. 6: eine Draufsicht auf eine optische Struktureinheit des in Figur 3 dargestellten Messkopfes,
- Fig. 7: eine Draufsicht auf eine Variante einer optischen Struktureinheit, welche zur Bestimmung von peripheren, intraokularen Distanzen, Längen oder Dicken verwendet werden kann,
- Fig. 8: eine Draufsicht auf eine weitere Variante einer optischen Struktureinheit,

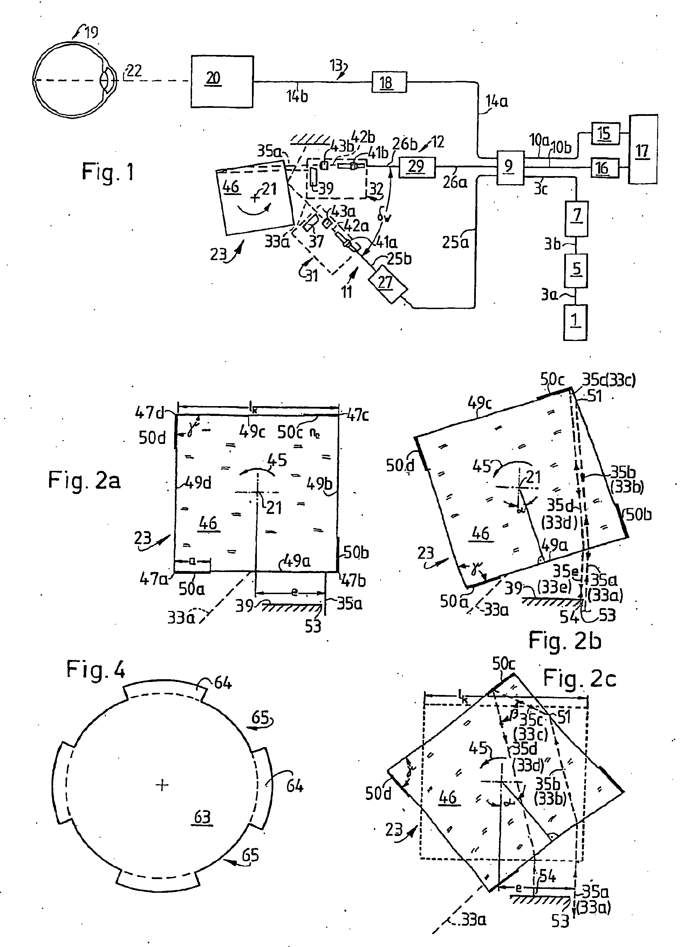

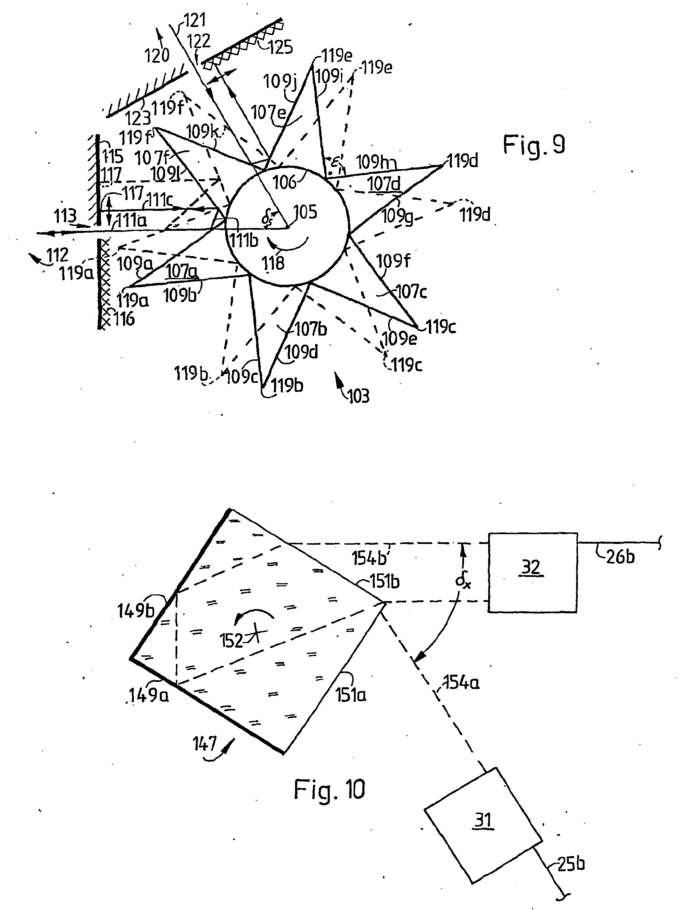

- Fig. 9: eine schematische Darstellung einer Variante zu dem in den Figuren 1, 2a, 2b und 2c dargestellten Weglängenvariationselement,
- Fig. 10: eine weitere Variante zu dem in den **Figuren 1, 2a, 2b** und **2c** dargestellten Weglängenvariationselement,

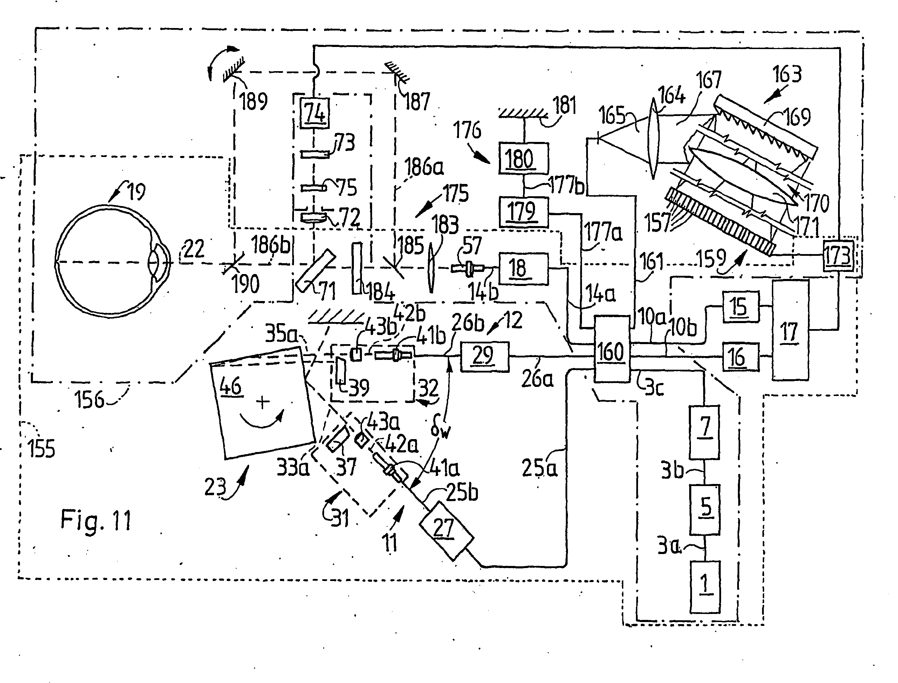

- Fig. 11: eine Variante zu der in **Figur 1** dargestellten Vorrichtung, bei der zusätzlich zur Ermittlung von Dicken bzw. Weglängen mit einem optischen Zeitraum-Kohärenztomographen eine Topographie eine zwei- oder eine dreidimensionale Tomographie mit einem optischen Spektralraumtomographen ermittelbar ist und

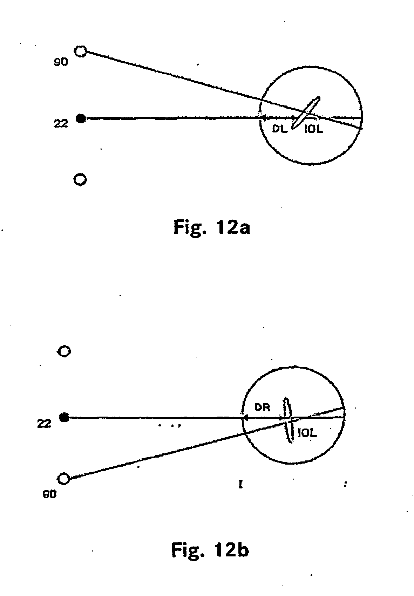

- Fig. 12 a-b: eine schematische Darstellung der Messung der Lage einer IOL.

### Wege zur Ausführung der Erfindung

In **Figur 1** ist ein Ausführungsbeispiel der erfindungsgemässen Vorrichtung mit einer "Michelson-Interferometer"-artigen optischen Anordnung dargestellt. Die Vorrichtung hat in der "Michelson-Interterometer"-artigen optischen Anordnung eine Strahlungsquelle 1, deren Strahlung eine kurze Kohärenzlänge hat. Die Kohärenzlänge ist wesentlich kürzer als die auszumessenden Dicken und Weglängen, und liegt im Bereich von 2 µm bis 100 µm. Wobei normalerweise in einem Bereich von 10 µm bis 20 µm gearbeitet wird. Je kürzer die Kohärenzlänge desto genauer kann gemessen werden. Man wird deshalb eine Strahlungsquelle 1 mit einer möglichst breitbandigen Ausgangsstrahlung wählen. Als Strahlungsquelle 1 kann eine Superlumineszenzdiode (SLD), eine Licht emittierende Diode (Light Emitting Diode LED), eine Weisslichtquelle bzw. eine Lichtquelle mit verstärkter spontaner Emission (Amplified Spontaneous Emission Lichtquelle (ASE), z. B. die Strahlung eines Dioden gepumpten Festkörpers) verwendet werden. Diese Strahlung wird in eine Monomode-Faser **3a** eingekoppelt.

Die Monomodefaser **3a** wird zu einem Strahlungsabschwächer **5** geführt. Der Strah-Iungsabschwächer **5** kann z. B. eine Verlustspleissung zwischen zwei nicht dargestellten Monomodefasem sein. Mit dem Strahlungsabschwächer **5** kann eine Abschwächung der von den unten beschriebenen Referenzarmen und vom Messarm in Richtung zur Strahlungsquelle **1** zurückreflektierten Strahlung vorgenommen werden. In die Strahlungsquelle **1** zurückreflektierte Strahlung könnte beispielsweise bei einer SLD, einer ASE oder bei einem Dioden gepumpten Festkörper das Emissionsverhalten negativ beeinflussen. Der Strahlungsabschwächer **5** dient somit zur Minimierung dieses negativen Einflusses. Vom Strahlungsabschwächer **5** wird die nun eventuell abgeschwächte Strahlung der Strahlungsquelle **1** in eine Monomodefaser **3b** zu einem Polarisationskontroller **7** geführt. Mit Hilfe des Polarisationskontrollers **7** wird der Polarisationszustand der Strahlung eingestellt, welche den Kontroller durchläuft. Der Strahlungsabschwächer **5** und der Polarisationskontroller **7** sind optional und sind für die erfindungsgemässe Vorrichtung nicht zwingend notwendig.

Die Strahlung der Strahlungsquelle **1** wird weiter mittels einer Monomodefaser **3c** auf einen 3 x 3 - Monomodefaserkoppler **9** geführt. Mit Hilfe des Monomodefaserkopplers **9** wird die Strahlung der Strahlungsquelle **1** in zwei Referenzarme **11** und **12** und in einen Messarm **13** aufgeteilt. Die beiden restlichen Ausgänge des 3 x 3 - Monomodefaserkopplers **9** sind mittels jeweils einem Strahlungsleiter **10a** und **10b** mit je einem Detektor **15** bzw. **16** verbunden, welcher seinerseits signaltechnisch mit einer Detektionselektronik **17** verbunden ist. Der Detektor **15** stellt Interferenzen der vom Messobjekt **19** reflektierten Strahlung mit reflektierter Strahlung im Referenzarm **12** fest. Der Detektor **16** misst Interferenzen der vom Messobjekt **19** reflektierten Strahlung mit reflektierter Strahlung im Referenzarm **11.**

Der Messarm **13** hat eine Monomodefaser **14a,** deren eines Ende mit dem Mohomodefaserkoppler **9** verbunden ist. Das andere Ende der Monomodefaser **14a** ist mit einem Polarisationskontroller **18** verbunden, von dem eine weitere Monomodefaser **14b** zu einem unten im Detail beschriebenen Messkopf **20** führt. Vom Messkopf **20** geht dann die Strahlung als Freiraumstrahl **22** zum Messobjekt **19** (hier einem Auge).

Die Referenzarme **11** und **12** haben entsprechend einer im oder am Messobjekt **19** auszumessenden Schichtdicke bzw. Länge eine unterschiedliche optische Grundweglänge. In beiden Referenzarmen **11** und **12** wirkt ein und dasselbe, um eine Elementachse **21** rotierbares Weglängenvariationselement **23.**

Die Referenzarme **11** und **12** sind über je eine Monomodefaser **25a** bzw. **26a** mit dem 3 x 3 - Monomodefaserkoppler **9** verbunden. Das jeweils dem Monomodefaserkoppler **9** abgewandte Ende der Monomodefaser **25a** bzw. **26a** ist mit einem Polarisationskontroller **27** bzw. **29** verbunden. Vom jeweiligen Polarisationkontroller **27** bzw. **29** führt je eine Monomodefaser **25b** bzw. **26b** zu einer optischen Einheit **31** bzw. **32,** welche die in der Monomodefaser **25b** bzw. **26b** u. a. geführte Strahlung jeweils in einen Freiraumstrahl **33a** bzw. **35a** verwandelt. Die beiden Freiraumstrahlen **33a** und **35a** werden unter einem gegenseitigen Versetzungswinkel δ**_{W}** (also nicht parallel) auf das Weglängenvariationselement **23** geführt und werden vom Weglängenvariationselement **23,** sofern dieses, wie unten beschrieben, eine bestimmte Drehposition hat, auf jeweils einen Spiegel **37** bzw. **39** in der optischen Einheit **31** bzw. **32** geführt und von diesem wieder über das Weglängenvariationselement **23** zurück in die Monomodefaser **25b** bzw. **26b** reflektiert. Der Versetzungswinkel δ**_{W}** ist bis eine Winkeltoleranz halb so gross wie ein Eckenwinkel γ des Weglängenvariationselements **23.** Die gewählte Winkeltoleranz beeinflusst die zur Verfügung stehende Weglängenvariationslänge. Je grösser die Winkeltoleranz gewählt wird, desto kleiner wird die Weglängenvariationslänge. Da in dem hier ausgesuchten Beispiel ein quadratischer Querschnitt des Weglängenvariationselements **23** gewählt worden ist, ist der Versetzungswinkel δ**_{w}** von 45 °. Das hier gewählte Weglängenvariationselement **23** hat einen Brechungsindex von 1,5 und eine Kantenbreite ℓ**ₖ** von beispielsweise 40 mm; man wird deshalb mit einer Winkeltoleranz von ± 5 ° arbeiten.

Jede optische Einheit **31** bzw. **32** hat eine Ferrule **41a** bzw. **41b** zum Fassen des dem Polarisationskontroller **27** bzw. **29** abgewandten Endes der Monomodefaser **25b** bzw. **26b.** Nach der Ferrule **41a** bzw. **41b** breitet sich die in der Monomodefaser **25b** bzw. **26b** geführte Strahlung als Freiraumstrahl **42a** bzw. **42b** aus und wird mittels einer Linseneinheit **43a** bzw. **43b** zum Freiraumstrahl **33a** bzw. **35a** kollimiert. Die Linseneinheit **43a** bzw. **43b** kann nun ein Achromat, eine Einzellinse oder auch ein Linsensystem sein. Der vom jeweiligen Spiegel **37** bzw. **39** rückreflektierte Strahl wird von der Linseneinheit **43a** bzw. **43b** über die Ferrule **41a** bzw. **41b** in die Monomodefaser **25b** bzw. **26b** eingekoppelt.

In den **Figuren 2a** bis **2c** ist das Weglängenvariationselement **23,** wie es u.a. in der EP 0 877 913 beschrieben ist, in unterschiedlichen Drehwinkelstellungen in einer vergrösserten Darstellung gezeigt. Die nachfolgenden Betrachtungen beziehen sich auf den in **Figur 1** gezeigten Referenzarm **12** und dessen Freiraumstrahl **35a** und den dort gezeigten Spiegel **39** in der optischen Einheit **32.** Das Element **23** ist durch einen nicht dargestellten Antrieb in Rotation, gemäss Pfeil **45,** versetzbar. Eine in den **Figuren 2a** bis **2c** dargestellte, senkrecht zur Elementachse **21** liegende Querschnittsfläche **46** des Elements **23,** in welcher der zweite Referenzstrahl **35b, 35c** und **35d** im Element **23** zu liegen kommt, weist vier Ecken **47a** bis **47d** mit einem Eckenwinkel γ von 90° auf und ist in dem hier ausgewählten Beschreibungsbeispiel quadratisch ausgebildet, d. h. das Element **23** ist ein gerader Zylinder mit quadratischer Grund- bzw. Querschnittsfläche. Die Rotationsachse **21** ist mit der Achse des Zylinders identisch. Jede Zylindermantelfläche **49a** bis **49d** des Elements **23** ist mit einer teilweisen Beschichtung **50a** bis **50d** versehen, welche derart ausgewählt ist, dass sie den im Element **23** befindlichen Teilstrahl **35c** bzw. den rückreflektierten Teilstrahlen **35d** des Referenzstrahls des zweiten Referenzarms **12** optimal reflektiert. Am Reflexionsort **51** des Strahls **35b** bzw. des rückreflektierten Strahls **35c** wird an der hier beispielsweise gezeichneten Wand **49b** keine Reflexionsbeschichtung benötigt, da hier Totalreflexion erfolgt. Die Beschichtungen **50a** bis **50d** beginnen jeweils an den Ecken (Kanten) **47a** bis **47d** und erstrecken sich über eine Distanz **a** (**Fig. 2a**) in die betreffende Seitenfläche **49a** bis **49d** hinein. Ausgehend von jeder Ecke **47a** bis **47d** ist jeweils nur eine der beiden anstossenden Seiten beschichtet, und zwar jeweils nur immer diejenige, auf der der reflektierte mit dem einfallenden Strahl einen spitzen Winkel β bilden, siehe hierzu insbesondere **Figur 2c****.**

Bei der in **Figur 2a** dargestellten Augenblicksstellung des rotierenden Elements **23** ist dessen Seite **49a** parallel zur Oberfläche des Spiegels (Reflektors) **39** stehend dargestellt. Der in das Element **23** eintretende Referenzstrahl **35a** ist derart geführt, dass er gerade an der in den **Figuren 2a, 2b** und **2c** dargestellten rechten Kante **53** des Spiegels **39** vorbeiführbar ist. Ein Abstand **e** von der Kante **53** des Spiegels **39** (bzw. **37)** ist geradeso gross gewählt, dass er nur geringfügig kleiner als die halbe Würfelkantenbreite ℓ**ₖ** ist. Bei dem hier gewählten Zahlenbeispiel mit einer Flächenbreite ℓ**ₖ** von 30 mm (gegenüber den oben genannten 40 mm), wird der Referenzstrahl **35a** in einem Abstand **e** von 13 mm von der zentrischen Rotationsachse **21** und in einem Abstand von etwa **1** mm von der rechten Kante **53** des Spiegels **39** (bzw. **37)** entfernt eingestrahlt.

**Figur 2b** zeigt das Element **23** gegenüber der Darstellung in **Figur 2a** in einer um einen beispielsweisen Winkel α von 20° verdreht. Der Strahl **35a** dringt unter Brechung als Strahl **35b** in das transparente Medium des Elements **23** ein und wird an der Fläche **49b** total reflektiert. Der auftreffende und der reflektierte Strahl **35b** und **35c** bilden miteinander einen stumpfen Winkel. Der reflektierte Strahl **35c** trifft auf die Innenseite der Fläche **50c** und wird dort durch den beschichteten Bereich **50c** als Strahl **35d** in Richtung Fläche **49a** parallel zum Strahl **35b** reflektiert. Die beiden Strahlen **35c** und **35d** bilden einen spitzen Winkel β miteinander. Der auf die Fläche **49a** auftreffende Strahl **35d** wird gebrochen, trifft als Strahl **35e** senkrecht auf den Spiegel **39** und wird dort unter einer annähernd 100%-igen Reflexion wieder in sich selbst zurück reflektiert, so dass der am Spiegel **39** reflektierte Strahl letztendlich nach Durchlaufen des Weglängenvariationselements **23** in dieselbe Richtung wie der eintretende Strahl **35a** dieses verlässt. Wie aus einem Vergleich der **Figuren 2b** und **2c** zu ersehen ist, wandert der Reflexionspunkt **54** des Strahls **35e** auf dem Spiegel **39** (bzw. 37) hin und her.

Die Weglängenänderung Δ**s** infolge der Rotation des Elements **23** setzt sich nun aus dem doppelten Weg der sich ändernden Weglängen der Strahlen **35a** bis **35e** zusammen. Für die Strahlen **35b** bis **35d** ist zu beachten, dass sich deren Weglängen durch den Brechungsindex **n** des Mediums, in dem sie laufen, verlängern. Gemäss **Figur 2c** ist die Weglängenänderung Δ**s** abhängig vom Drehwinkel α, vom Abstand des Strahleintritts **e,** vom Brechungsindex n und der Flächenbreite ℓ**ₖ** der Flächen **49a** bis **49d.** Die Berechnung der Weglängenänderung Δ**s** über dem Drehwinkel α ist in der EP 0 877 913 abgeleitet und ergibt über einen Drehwinkel von 45 ° eine im Wesentlichen lineare Weglängenänderung.

Um die **Figuren 2a** bis **2c** nicht zu überladen, ist lediglich der Freiraumstrahl **33a** des zweiten Referenzarmes **11** dargestellt. D. h. der Strahlverlauf der nicht im Detail gezeichneten Strahlen **33a** bis **33d** entspricht dem Strahlverlauf der Strahlen **35a** bis **35d** unter einem gleichen Auftreffwinkel auf die entsprechende Zylindermantelfläche **49a** bis **49d** für den Strahl **33a.** Ein Strahlverlauf der Strahlen **33a** bis **33d** in der in **Figur 2a** gezeigten Position des Elements **23** ist mit den Strahlverlauf der Strahlen **35a** bis **35d** in der **Figur 2c** gleichzusetzen. Er ergibt sich nach einer Drehung um den halben Eckenwinkel γ/2; die entsprechenden Teilstrahlen sind deshalb in runde Klammem gesetzt. Der Strahlenverlauf der Strahlen **35a** bis **35d** in **Figur 2c** würde dann einem Strahlenverlauf der Strahlen **33a** bis **33d** in **Figur 2a** entsprechen. Ein in der in **Figur 2b** gezeigten Elementposition auftreffender Strahl **33a** wird nicht mehr zum entsprechenden Spiegel **37** zurück reflektiert.

Die oben angeführten Daten beziehen sich auf ein Material des Weglängenvariationselementes, das bei der Strahlung der Strahlungsquelle einen Brechungsindex von etwa 1,5 hat. Wird ein Material mit höherem Brechungsindex verwendet, so kann die Beschichtungsbreite **a** der reflektierenden Beschichtungen **50a** bis **50d** verringert werden, damit die zeitlichen Messbereiche der Referenzarmen auseinander gehalten werden können.

Die optischen Längen der Referenzarme **11** und **12** können derart ausgebildet werden, dass dadurch kein nahtloses örtliches Aneinanderfügen der zwei von den Referenzarmen **11** und **12** zur Verfügung gestellten Messbereiche entsteht. Wird der für den hinteren Augenabschnitt zuständige Referenzarm genug lang ausgebildet, so beginnt der Messbereich des für den hinteren Augenabschnitt zuständigen Referenzarmes in einer Position, die um eine vorgegebene Weglänge näher an der Retina liegt als die am tiefsten im Auge **19** liegende Position des für den vorderen Augenabschnitt zuständigen Referenzarmes. Mit dieser Massnahme entsteht eine Scanlücke zwischen dem vorderen und dem hinteren Tiefenscan. D. h., es gibt eine vorgegebene (konstruktiv einstellbare) Strecke zwischen einem vorderen Augenabschnitt und einem hinteren Augenabschnitt, auf der keine reflektierenden Strukturen ausgemessen werden können.

Diese Scanlücke wird man beim menschlichen Auge in der Grössenordnung von ca. 4 mm bis 10 mm wählen. Bei der Aufnahme des Tiefenscans von der Cornea über die Augenlinse bis zur Netzhaut führt eine derartige Scanlücke zu einem vernachlässigbaren minimalen Informationsverlust, weil diese Scanlücke in einen Augenbereich gelegt worden ist, in dem bei einer überwiegenden Anzahl von Patienten bzw. Normalprobanten keine auszumessende Augenstruktur vorhanden ist; die Scanlücke ist hinter die Linsenfläche im vordersten Teil des Glaskörpers gelegt. Ein Vorteil der Scanlücke besteht darin, dass das um eine Achse rotierende Weglängenvariationselement **23** eine um die der Scanlücke entsprechende Weglänge kürzere Scantiefe zur Verfügung stellen muss. Es kann deshalb ein Weglängenvariationselement **23** mit einer kürzeren Seitenlänge **49a** bis **49d** verwendet werden, was eine höhere Empfindlichkeit bei gleicher Scangeschwindigkeit zur Folge hat.

Die in den beiden Referenzarmen **11** und **12** sowie im Messarm **13** angeordneten Polarisationskontroller **18, 27** und **29** dienen zur Anpassung der Strahlungspolarisationen in den Armen **11, 12** und **13.** Die optischen Weglängendifferenzen der beiden Referenzarme **11** und **12** werden immer so eingestellt, dass der Unterschied der Grundlaufzeiten der beiden Referenzarme **11** und **12** der Hälfte der maximal auszumessenden Längen, hier beispielsweise der Augenlänge, entspricht.

Sind die Differenzen der beiden Referenzarme grösser, können auch längere Distanzen ausgemessen werden, jedoch mit der Einschränkung, dass Reflexionen in einem dazwischen liegenden Bereich nicht detektiert werden können.

Zeitlich gesehen, fallen die beiden Interferenzen entsprechend der Rotationsgeschwindigkeit und der Anzahl Zylindermantelflächen des Weglängenvariationselements 23 sehr schnell hintereinander an. Da bei Abständen, Dickenmessungen, ... beim Stand der Technik immer zwei zeitlich gestaffelte Messungen vorzunehmen waren, liegt nun bei der Erfindung das Messergebnis derart schnell vor, dass Ortsverschiebungen des auszumessenden Gegenstands die Messgenauigkeit nur unwesentlich beeinflussen. Der gerade geschilderte Vorteil ist bei Längenmessung an den Augen von Kindern, welche in der Regel nur schwer still zu stellen sind, von grossem Nutzen.

Den in **Figur 1** nicht näher beschriebenen Messkopf **20** zeigt **Figur 3** detaillierter. In den Messkopf **20** tritt die Monomodefaser **14b** des Messarms **13** ein. Am Ende der Faser **14b** ist zur Halterung eine so genannte Ferrule **57** angeordnet. Die in der Faser **14b** geführte Strahlung tritt aus der Ferrule **57** entsprechend dem Faserkem mit einem vorgegebenen Öffnungswinkel aus und wird mit einer Fokussiereinheit **58,** welche eine Linseneinheit **59** aufweist, auf eine Vorderfläche einer Schicht, deren Dicke bestimmt werden soll, fokussiert. Als Schicht kann in dem hier gewählten Beispiel die Hornhaut des Auges (Messobjekt **19)** vorgesehen sein. Wie bereits oben erwähnt, ist es bei der Schichtdickenbestimmung von Vorteil, wenn die Messstrahlung sowohl auf die Vorderwie auch anschliessend auf die Rückseite fokussierbar ist. Soll nun auf die Rückseite der Schicht fokussiert werden, so wird zwischen der Ferrule **57** und der Linseneinheit **59** eine planparallele Platte **61** mit vorgegebenem Brechungsindex n und vorgegebener Dicke **d,** als Teil der Fokussiereinheit **58** eingeschoben. Durch diese Platte **61** ergibt sich eine Bildversetzung **v** des Faserendes in der Ferrule **57** und somit auch eine Verschiebung des Fokuspunktes in der Tiefe des Objektes. Die Bildversetzung wird nun in Zusammenhang mit den optischen Abbildungsdaten der Linseneinheit **59** durch eine geeignete Wahl der Dicke **d** und des Brechungsindexes **n** der planparallelen Platte **61** derart gewählt, dass das Faserende in der Ferrule **57** auf der Schichtrückseite, d. h. hier auf der Netzhaut (Augenrückseite) abgebildet wird. Ein Einbringen der Platte **61** in den Messstrahl **22** wird synchron mit dem oben beschriebenen Wegvariationselement **23** derart vorgenommen, dass eine Weglängenvariation gerade mittels demjenigen Referenzarm erfolgt, der für eine Bestimmung der Schichtvorderseite (→ keine Platte, d. h. herausgezogene Platte) bzw. für die Schichthinterseite (→ eingeschobene Platte) zuständig ist.

Da nun ein Hin- und Herschieben einer planparallelen Platte **61** mit einigen Schwierigkeiten verbunden ist, wird vorgeschlagen, statt dessen eine in **Figur 4** dargestellte, rotierende Scheibe **63** mit vorstehenden Randkreisringteilstücken **64** ("Zahnung"), deren Scheibenseiten planparallel und transparent ausgebildet sind, zu verwenden. Die von der Ferrule **57** ausgehende Messstrahlung durchläuft nun bei der Messung der Schichtvorderseite einen der Zwischenräume **65** zwischen den Randkreisringteilstücken **64** und beim Ausmessen der Schichtrückseite einen der Randkreisringteilstücke **64.** Die Drehbewegung der Scheibe **63** ist mit der Rotation des Weglängenvariationselements **23** über einen nicht dargestellten Antrieb gekoppelt.

Wie bereits eingangs erwähnt, ist es von Vorteil, wenn neben einer Bestimmung von Schichtdicken, insbesondere beim menschlichen Auge, zudem noch die Topographie als weiterer geometrischer Wert der betreffenden Schicht ermittelt werden kann. Zur Ermittlung der Topographie von Oberflächen kann der Messkopf **20,** wie ebenfalls in **Figur 3** gezeigt, zusätzliche Komponenten enthalten.

Vor der Oberfläche, deren Topographie, hier der Cornea **69**, ermittelt werden soll, wird eine optische Struktureinheit **70** mit Bereichen unterschiedlicher Leuchtdichte angeordnet. Die Reflexionen dieser Struktur auf der Hornhaut werden abgebildet und diese Abbildung ausgewertet. Da die Geometrie der Struktur bekannt ist, kann aus der Geometrie in der Abbildung, wie in der Einleitung ausgeführt ist, auf die Oberflächentopographie geschlossen werden.

Der Messstrahl **22** für die Schichtdickenbestimmung wird vorzugsweise mittig durch die Struktureinheit **70** geführt. Hierzu wird der Strahl **22** nach der Linseneinheit **59** mit einem wellenlängen-selektiv arbeitenden Spiegel **71** umgelenkt. Damit dieser Spiegel **71** wirken kann, werden für die Messstrahlung zur Schichtdickenbestimmung und für die Strahlung der Struktur unterschiedliche Wellenlängen verwendet. Der Spiegel **71** hat somit eine Beschichtung, welche reflektierend für die Messstrahlung ist, jedoch (annähernd) verlustlos transmittierend für die "Strukturstrahlung". In einer rückwärtigen Verlängerung definiert der Strahl **22** eine optische Achse **68** für die Abbildung der Reflexstrukturen auf der Corneaoberfläche **69.**

Zur Abbildung der von der Corneaoberfläche **69** reflektierten Struktur wird eine weitere Linseneinheit **72** mit nachgeschalteter Blende verwendet, welche die Comeaoberfläche **69** auf die Bildebene eines Kamerachips **73** abbildet. Das Linsensystem **72** ist zwischen dem Spiegel **71** und dem Kamerachip **73** angeordnet. Damit die Messstrahlung des Strahls **22** die Topografieauswertung nicht stört, ist zwischen dem Spiegel **71** und dem Kamerachip **73** ein Sperrfilter **75** für die Messstrahlung angeordnet. Die Messstrahlung kann für den Patienten gleichzeitig als Fixationsstrahlung zur Ruhigstellung des Auges verwendet werden. Der Kamerachip **73** ist mit einer Auswerteeinheit **74** verbunden, welche zudem auch mit der Detektionseinheit **17** verbunden ist. Die Auswerteeinheit **74** ist Teil der bereits oben und noch nachfolgend beschriebenen Vorrichtung und dient zur Verarbeitung, Übermittlung und Darstellung sämtlicher Messdaten.

Eine beispielsweise Ausbildung **79** einer optischen Struktureinheit **70** ist in **Figur 6** dargestellt. Die Struktureinheit **79** hat 16 LEDs **80a** und **80b,** welche in zwei konzentrischen Ringen **82a** und **82b** zu je acht LEDs angeordnet sind. Die Winkeldifferenz zwischen zwei aufeinander folgenden LEDs beträgt 45 °. Vorzugsweise strahlen diese LEDs im nicht oder kaum sichtbaren Infrarotbereich. In diesem Fall wird das Auge des Patienten nicht von unnötigen Lichtreizen abgelenkt. Es können auch mehr als zwei Ringe und auch mehr als acht LEDs pro Ring verwendet werden.

Zwei LEDs, deren geometrische Verbindungslinie durch das Zentrum **81** der Struktureinheit geht, erlauben die Messung eines mittleren Krümmungsradius' zwischen der Verbindungslinie der beiden LED-Abbildungen (=Messung eines Homhautmeridians). Aus der Vielzahl von Homhautkrümmungsradien der Meridiane interessieren bei der Anpassung der Brechkraft einer Intraokularlinse vor allem der steilste und der flachste Meridian bzw. der Mittelwert von beiden. Die Richtungen des steilsten und des flachsten Meridians müssen nicht unbedingt mit der Richtung eines der gemessenen Krümmungsradien übereinstimmen. Der flachste Krümmungsradius kann auch noch ein bisschen flacher sein als der grösste der gemessenen Krümmungsradien und der steilste Krümmungsradius kann auch noch ein bisschen kleiner als der steilste der gemessenen Krümmungsradien sein.

Es müssen wenigstens fünf LEDs vorhanden sein, damit der steilste und der flachste Meridian sowie den Achswinkel des flachen Meridians berechnet werden können. Bei der Messung der Krümmungsradien werden alle LEDs gleichzeitig ein- und ausgeschaltet.

Zwischen zwei beliebigen LEDs, deren Verbindungslinie nicht unbedingt durch das Zentrum **81** gehen müssen, kann ein mittlerer Krümmungsradius berechnet werden. Dieser mittlere Krümmungsradius tritt auf der Verbindungslinie zwischen den zwei entsprechenden LEDs auf. Mit der Anzahl der vorhandenen LEDs nimmt die Anzahl der messbaren Krümmungsradien pro Oberflächeneinheit zu. Mit einer genügend grossen Anzahl von LEDs kann eine Homhauttopographie berechnet werden. Je grösser die Anzahl Messpunkte (=LED) desto genauer kann eine Topografie ermittelt werden. Es kann eine beliebige Anzahl von konzentrischen Ringen und eine beliebige Anzahl von LEDs pro Ring für die Messung der Hornhautradien verwendet werden. Auch kann eine beliebige Anzahl von konzentrischen Ringen und eine beliebige Anzahl von LEDs pro Ring als Fixationsstrahler verwendet werden. Nach oben ist die Anzahl der verwendeten LEDs lediglich durch den Durchmesser des Rings und die Abmessungen der LEDs beschränkt.

Der Messstrahl **22** der Interferometeranordnung kann auch zur Distanzmessung zwischen der Vorrichtung und der Hornhautvorderfläche als Messobjekt **19** verwendet werden. Diese Distanz wird benötigt, wenn die Abstände zwischen den verschiedenen auf dem Kamerachip **73** abgebildeten LED-Flecken abhängig sind von der Distanz zwischen den LEDs und der auszumessenden Hornhautvorderfläche. Diese Distanzabhängigkeit tritt vor allem dann wesentlich in Erscheinung, wenn z. B. aus Kostengründen für die Linseneinheit **72** keine telezentrische Linse verwendet wird. Die Distanzmessung erlaubt dann eine exaktere Berechnung der Homhautkrümmungsradien als es ohne Distanzkenntnis möglich wäre. Die zu messende Distanz zur Hornhautvorderfläche wird entweder unter Verwendung des Referenzarmes **11** oder **12** bestimmt.

Da eine Packungsdichte von LEDs limitiert ist, (jede einzelne LED hat eine vorgegebene geometrische Ausdehnung), kann eine Lochmaske verwendet werden, die von hinten mit mehreren LEDs bestrahlt wird. Der Durchmesser der Löcher der Lochmaske kann wesentlich kleiner gewählt werden als die Ausdehnung jeder LED. Hierdurch kann eine grössere Anzahl von Leuchtpunkten erhalten werden. Die Lochmaske muss nicht unbedingt kreisrunde Löcher aufweisen; es können auch Linien vorhanden sein. Diese Linien können als konzentrische Kreise sowie als eine Kombination von konzentrischen Kreisen und diese schneidende Strahlen ausgebildet sein.

Es kann auch, wie in **Figur 3** angedeutet ist, eine Streuscheibe **83** vor den LEDs platziert sein. Die Streuscheibe **83** erzeugt auf der auszumessenden Oberfläche einen Leuchtdichtefleck, dessen laterale Ausdehnung sich durch den Abstrahlwinkel der LED, der Dicke der Streuscheibe und dem Abstand zwischen Streuscheibe **83** und der betreffenden LED ergibt. Die Streuscheibe **83** ist nicht zwingend notwendig. Ist keine Streuscheibe **83** vorhanden, hat die auf dem Kamerachip abgebildete LED einen kleineren Durchmesser. Ein Nachteil von zu kleinen abgebildeten Durchmessern ist u. U. eine verringerte Genauigkeit der Bestimmung eines Schwerpunkts des abgebildeten Flecks. Ein nur wenige Pixel aufweisender Fleck kann in der Regel weniger genau ausgemessen werden als ein eine grössere Anzahl Pixel aufweisender Fleck. Wird eine Streuscheibe **83** verwendet, können LEDs mit erhöhter Leuchtdichte verwendet werden, da dann die Bestrahlungsstärke auf dem Auge geringer ist.

Bei der in **Figur 6** gezeigten Struktureinheit **79** sind nicht oder kaum sichtbare Strahlung aussendende LEDs **80a** und **80b** verwendet worden. In **Figur 7** ist nun eine optische Struktureinheit **87** dargestellt, bei der zwischen vorzugsweise infrarot leuchtenden, radial angeordneten LEDS **89a** und **89b** weitere LEDs **90** angeordnet sind, welche im Sichtbaren leuchten. Wird nun eine dieser LEDs **90** angeschaltet, so wird dem Patienten ein peripherer Fixationspunkt angeboten. Fixiert nun der Patient auf diesen Lichtreiz, bewegt er die Augenachse auf diesen Punkt, wobei dann eine interferometrische Messung mit dem Messstrahl **22** von peripheren Intraokularen Distanzen, Längen oder Dicken möglich ist. Im Gegensatz zu bekannten Vorrichtungen muss keine Verschwenkung der Anordnung vorgenommen werden.

Werden vier periphere Messungen mit dem Messstrahl **22** mit Hilfe von vier verschiedenen, zeitlich nacheinander eingeschalteten sichtbaren LEDs **90** (Fixations-LEDs) hintereinander durchgeführt, so lässt sich daraus die Verkippung einer implantierten intraokularen Linse (IOL) messen. Die **Figur 12** zeigt nur die Messung der horizontalen Verkippung mit zwei zeitlich nacheinander eingeschalteten horizontalen LEDs **90.** Kommt als Resultat der zwei gezeigten Messungen heraus, dass die Strecke DL ("Distanz links") nicht gleich der Strecke DR ("Distanz rechts") ist, so ist die IOL horizontal verkippt. Für die Messung der vertikalen Verkippung müssen zwei vertikale LEDs **90** zeitlich nacheinander eingeschaltet werden. Mit Hilfe der Kenntnis der Verkippung erhält der Arzt die Entscheidungsgrundlage, um eine verkippte intraokulare Linse neu auszurichten

In **Figur 8** ist eine weitere Ausführungsvariante **91** zu den optischen Struktureinheiten **70** und **87** dargestellt. Mit dieser Struktureinheit **91** kann nicht nur die Hornhautvorderfläche, sondern auch die Hornhautrückfläche, sowie die Linsenvorderfläche und die Linsenrückfläche ausgemessen werden. In dem hier gezeigten Beispiel sind in einer zu einem Zentrum **93** radialen sternförmigen Anordnung von acht Ästen **94a** bis **94h** jeweils vier auf koaxialen Kreisen **95a** bis **95d** angeordnete LEDs **97** vorhanden. Die LEDs eines Astes **94a** bis **94h** sind jeweils von innen nach aussen laufend mit dem Buchstaben des Astes zusätzlich gekennzeichnet. Eine im Ast **94a** zu innerst liegende LED hat somit die Kennzeichnung **94a₁** und eine zu äusserst liegende LED die Kennzeichnung **97a₄.** Über jedem der Äste **94a** bis **94h** ist jeweils eine Zylinderlinse **99** angeordnet. Die acht Zylinderlinsen **99** fokussieren die LEDs **97** auf einen Bereich zwischen Hornhaut und Augenlinse. Werden nun eine Reihe von LEDs, z. B. die LEDs **97a₄** bis **97a₁** und **97e₁** bis **97e₄** eingeschaltet, welche auf einer das Zentrum **93** schneiden Verbindungslinie liegen, so entsteht ein Lichtspalt auf dem Auge. Mit einer kurzen Zeitverzögerung werden dann die LEDs **97a₄** bis **97a₁** und **97e₁** bis **97e₄** ausgeschaltet und dann die LEDs **97b₄** bis **97b₁** und **97f₁** bis **97f₄** eingeschaltet. Anschliessend werden die LEDs **97b₄** bis **97b₁** und **97f₁** bis **97f₄** aus- und die LEDs **97c₄** bis **97c₁** und **97g₁** bis **97g₄** eingeschaltet usw. Hierdurch entsteht ein um das Zentrum **93** rotierender Lichtspalt. Eine analog zu der in **Figur 3** zentral angeordnete Kamera betrachtet dann eine Abfolge der Lichtspalte, die von den Zylinderlinsen **99** in das Auge projiziert werden. Zwei nicht dargestellte, peripher angeordnete Kameras (z. B. eine vertikal, eine horizontal angeordnet) erlaubt eine zusätzliche Messung der Lichtspalte. Aus den auf dem Kamerachips erscheinenden Positionen der Lichtspalte lässt sich die Topographie der Hornhautrückfläche, der Vorderkammertiefe (dreidimensional), die Topographie der Linsenvorderfläche und der Linsenrückfläche, die Linsendicke (dreidimensional) und die Linsentrübung berechnen. Die oben beschriebene Interferometeranordnung (siehe **Figur 3)** wird verwendet, um die Distanz zu messen, die bei der Erzeugung der Kamerabilder vorgelegen hat. Die von der Kamera dann gemessenen Oberflächen werden noch mit der gemessenen Distanz korrigiert.

Gegenüber der Darstellung in **Figur 8** kann auch eine andere Anzahl von LEDs verwendet werden. Zwischen den LEDs und den Zylinderlinsen kann zusätzlich ein nicht dargestellter, vorzugsweise linear wirkender Diffusor angeordnet werden, um die Lichtflecke der punktförmigen LEDs ineinander fliessen zu lassen. Es sind hierdurch homogen ausgeleuchtete Lichtspalte erzeugbar.

Anstatt die LEDs nacheinander ein- und auszuschalten, kann auch ein rotierender mechanischer Spalt über sämtlichen eingeschalteten LEDs bewegt werden.

Anstelle der acht in **Figur 8** gezeigten Zylinderlinsen **99** kann auch lediglich ein, nicht dargestelltes, zwei miteinander fluchtende Zylinderlinsen aufweisendes Zylinderlinsenpaar, welches um das Zentrum **93** rotiert, verwendet werden.

Die in **Figur 4** dargestellte, Kreisringteilstilcke aufweisende rotierende Scheibe **63** dient zur Umschaltung der Fokussierung der Messstrahlung auf zwei unterschiedliche Fokuspunkte. Soll nun eine Umschaltung auf mehr als zwei Fokuspunkte erfolgen, so müssten je nach Anzahl umzuschaltender Fokuspunkte die Segmente eine entsprechende Anzahl planparalleler Stufungen aufweisen. Eine derartige Stufung ist nur schwer und aufwendig herstellbar. Um eine derartige Stufung zu erhalten, wird man deshalb mehrere mit entsprechenden kreisringteilstückartigen Ausnehmungen versehene planparallele Scheiben **131** und **132** zu einer Fokuspunktverschiebeeinrichtung **130,** wie in den **Figuren 5a** bis **5d** dargestellt, aufeinander legen. Die in **Figur 5a** skizzierte Scheibe **131** entspricht nun beispielsweise in ihrer Kontur der in **Figur 4** gezeigten rotierenden, planparallelen Scheibe **63,** wobei die Dicke eine andere sein könnte. Die Scheibe **131** hat beispielsweise bei einer Augenuntersuchung den Zweck während beispielsweise des Wirkens des Referenzarmes **11,** den Fokus von der Homhautoberfläche zur Retina zu verschieben und weist die Dicke **b₁** auf. Auf die Hornhaut würde immer dann fokussiert, wenn der Messstrahl durch die freien Kreisringsteilstücke **133** hindurchtritt und auf die Retina würde fokussiert, wenn der Strahl durch die transparenten Materialkreisrandringteilstücke **135** hindurchtritt. Vorgängig gesagtes gilt, sofern der Abstand Ferrule **57 -** Linse **59** in **Figur 3** grösser ist als die Brennweite der Linse. Im umgekehrten Fall, d. h. bei einem Abstand der Ferrule **57,** welcher kleiner ist als die Brennweite der Linse, wird die Retina gemessen, wenn der Messstrahl durch das freie Kreisrandringteilstück hindurchtritt. Die Hornhaut wird gemessen, wenn der Strahl durch das transparente Kreisrandringteilstück hindurch tritt. Da das Retina-Signal, insbesondere bei Caterakt-Patienten schwächer ist als bei einer normalen Retina, wird man bei Caterakt-Patienten den letzt genannten Aufbau bevorzugt verwenden.

Es sind je vier Kreisringteilstücke **133** und **135** vorhanden, welche alternierend angeordnet sind. Ein Zentriwinkel ϕ jedes Kreisringteilstückes **133** bzw. **135** beträgt 45 °. Der äussere Kreisbogen des Kreisringteilstückes **133** hat einen Radius **r₁** und das Kreisringteilstück **135** einen um Δ**r** vergrösserten, äusseren Kreisbogen mit einem Radius **r₂.** Δ**r** ist so gross gewählt, dass der Messstrahl **22** gut Platz hat. Es liegen somit immer zwei gleiche Kreisringteilstücke **133** bzw. **135** diagonal einander gegenüber.

Die zweite, in **Figur 5b** skizzierte Scheibe **132** hat dann die Aufgabe, während des Wirkens des Referenzarmes **12,** den Fokuspunkt in die Augenlinse zu legen und weist die Dicke **b₂** auf. Die Dicke **b₂** der rotierenden planparallelen Scheibe **132** unterscheidet sich von der Dicke **b₁** der Scheibe **131.** Die Scheibe **132** hat zwei einander diagonal gegenüberliegende transparente Materialkreisrandringteilstücke **137,** welche jeweils einen Zentriwinkel ρ**₁** von 45° und einen Radius **r₂** aufweisen. Die zwischen den Kreisringteilstücken **137** liegenden beiden freien Kreisringstücke **139** haben somit einen Zentriwinkel ρ**₂** von 135° und einen Radius **r₁.**

Beide Scheiben **131** und **132** werden, wie in **Figur 5c** angedeutet, derart übereinander gelegt, dass einerseits ihre Rotationsachsen **141** und **142** fluchten und andererseits jeweils die transparenten Materialkreisrandringteilstücke **137** hinter zweien der vier freien Kreisringteilstücke **133** zu liegen kommen.

In **Figur 5c** befindet sich der Messstrahl **22** gerade in den freien Kreisringteilstücken **133** und **137** (Bereich **A**), wodurch bei dem obigen Beispiel einer Augenuntersuchung eine Fokussierung auf die Hornhaut gegeben ist. Wird die Fokuspunktverschiebeeinrichtung **130** der Fokussiereinheit **58** in Richtung des Rotationspfeils **143** in **Figur 5c** weiter gedreht, so passiert der Messstrahl **22** das transparente Materialkreisrandringteilstück **135** mit der grossen Dicke **b₁** (Bereich **B**), wodurch sich eine Fokussierung auf die Retina ergibt. Nach einer weiteren Verdrehung transmittiert der Messstrahl **22** das transparente Materialkreisrandringteilstück **137** mit der dünnen Dicke **b₂** (Bereich **C**), wodurch der Fokus in die Augenlinse gelegt wird und deren Dicke ermittelt wird. Wird zum Bereich **D** weitergedreht, transmittiert der Messstrahl **22** wieder Material mit der Dicke **b₁** und wird somit auf die Retina analog zum Bereich **B** fokussiert. Im Bereich **E** transmittiert der Messstrahl **22** lediglich Luft und wird somit auf die Hornhaut fokussiert. Im Bereich **F** wird wieder auf die Retina fokussiert, im Bereich **G** die Dicke der Augenlinse ausgemessen und im Bereich **H** wieder auf die Retina fokussiert. Mit der hier beschriebenen Anordnung kann somit die Augenlänge und die Dicke der Augenlinse in einem Messverfahren ermittelt werden.

Die oben geschilderte Fokuspunktverschiebeeinrichtung **130** arbeitet mit zwei rotierenden Scheiben, welche freie Bogenstücke aufweist. Die jeweilige Lage des Fokuspunktes wird durch die jeweilige Dicke des gerade transmittierten Materials und dessen Brechungsindexes bestimmt. Eine Fokuspunktverschiebeeinrichtung kann nun auch derart aufgebaut werden, dass sich die Dicken der einzelnen Scheiben zu einer Gesamtdicke addieren. Es ist dann jedoch darauf zu achten, dass keine Luftspalte zwischen den Platten entstehen, welche unerwünschte Reflexionen bewirken würden. Statt zwei können jedoch auch mehr Scheiben verwendet werden. Auch kann eine stärkere Unterteilung vorgenommen werden, so dass statt der oben geschilderten drei Fokusorte weitere Fokusorte entstehen.

Anstelle der lediglich beiden Referenzarme **11** und **12** können selbstverständlich auch drei, vier und mehr Referenzarme unter entsprechenden gegenseitigen Winkeln verwendet werden. Die einzelnen Referenzarme können auch mit entsprechenden, mit der Rotation des Wegvariationselements synchronisierten Strahlunterbrechern vor dem einzigen Wegvariationselement zu- und abgeschaltet werden.

Anstelle des in der EP 0 877 913 beschriebenen und in den **Figuren 1, 2a, 2b** und **2c** dargestellten Weglängenvariationselements **23** können auch mit einigen Änderungen die in den **Figuren 1** bis **3** der DE 34 46 014 dargestellten Weglängenvariationselemente verwendet werden. Bei Verwendung des Weglängenvariationselements **23** wird dieses von den Referenzstrahlen durchstrahlt. Bei dem in **Figur 9** dargestellten Weglängenvariationselement **103** hingegen werden die Referenzstrahlen an seiner Oberfläche reflektiert; die Referenzstrahlen treten nicht in das Weglängenvariationselement **103** ein. Das Weglängenvariationselement **103** ist ebenfalls als um eine Achse **105** rotierender Körper ausgebildet. Auf einem geraden Kreiszylinder **106** sind sechs gleichschenklige Dachkantprismen **107a** bis **107f** mit verspiegelten Aussenflächen **109a** bis **109ℓ** angeordnet. Jeweils an der Basis der Dachkantprismen **107a** bis **107f** auf dem Kreiszylinder **106** zusammentreffende Aussenflächen **109a/109ℓ, 109c/109b, 109e/109d, 109g/109f, 109i/109h** und **109k/109j** schliessen einen rechten Winkel ε miteinander ein. Ein freier Strahl **111a** eines zum Referenzarm **12** analog ausgebildeten Referenzarms **112** wird durch eine Öffnung **113** geführt, wobei in **Figur 9** oberhalb der Öffnung **113** ein zum Strahl **111a** senkrecht verlaufender Spiegel **115** angeordnet ist, welcher annähernd 100%-ig reflektierend für die Strahlung des Strahls **111a** ausgebildet ist. Unterhalb der Öffnung **113** ist ein Absorber **116** für die Strahlung des Strahls **111a** angeordnet. Der Strahl **111a** trifft nun in der in **Figur 9** dargestellten Stellung des Weglängenvariationselements **103** auf die Aussenfläche **109a** und wird von hier als Strahl **111b** zur Aussenfläche **109ℓ** reflektiert und von dieser parallel zum Strahl **111a** als Strahl **111c** auf den Spiegel **115** geführt und in sich selbst zurückreflektiert, so dass er als reflektierter Strahl wieder durch die Öffnung **113** hindurch tritt. Aufgrund des rechten Winkels ε wird der auf den Spiegel **115** auftreffende Strahl immer in sich selbst zurückreflektiert, auch wenn das Weglängenvarlationselement **103** um einen Winkel, wie in **Figur 9** gestrichelt angedeutet, in Richtung des Pfeils **118** weiter gedreht worden ist. Der Auftreffort **117** des Strahls **111c** wandert von der Öffnung **113** in **Figur 9** nach oben weg bis der Strahl **111a** auf die Spitze **119a** des Dachkantprismas **107a** fällt. Dreht das Weglängenvariationselement **103** weiter, so trifft der Strahl **111c** von der Öffnung **113** entfernt auf den Absorber **116** und wandert dann in Richtung zur Öffnung **113;** d. h. es erfolgt jetzt keine Strahlreflexion.

Ein Strahl **121** eines analog zum Referenzarm **11** ausgebildeten zweiten Referenzarms **120** trifft durch eine Öffnung **122** tretend in dem Augenblick, in dem der Strahl **111a** auf die Spitze **119a** trifft, auf eine Spitze **119f** des Dachkantprismas **107f** und wird nach einem kurzen Weiterdrehen von der Fläche **109ℓ** auf die Fläche **109a** reflektiert und von dieser auf einen Spiegel **123** und von dort wieder in sich selbst zurück reflektiert, so dass der reflektierte Strahl wieder durch die Öffnung **122** hindurch tritt. Hat das Weglängenvariationselement **103** soweit weiter gedreht, dass der Strahl **121** auf die Fläche **109a** trifft, fällt der dann an der Fläche **109** ℓ reflektierte Strahl wieder auf einen Absorber **125,** wie in **Figur 9** dargestellt, schräg oberhalb der Öffnung **122.** Die beiden einfallenden Strahlen **111a** und **121** schliessen einen Versetzungswinkel δ**ₛ** miteinander ein. Der Versetzungswinkel δ**ₛ** entspricht bis auf eine Toleranz dem Zentriwinkel jeweils benachbarter Spitzen der Dachkantprismen. Je grösser auch hier die Toleranz gewählt wird desto kleiner ist die Weglängenvariation.

Wenn der Strahl **111c** auf den Reflektor **115** trifft, trifft der durch Reflexionen umgelenkte Strahl **121** auf den Absorber **125.** Trifft der umgelenkte Strahl **119** auf den Spiegel **123,** so trifft der Strahl **111c** auf den Absorber **116.**

Anstatt das Weglängenvariationselement **103** mit sechs Dachkantprismen **107a** bis **107f** auszubilden, kann auch eine andere Anzahl von Dachkantprismen verwendet werden: Auf dem Kreiszylinder müssen jedoch immer benachbarte Flächen unter einem Winkel von 90 ° zusammentreffen.

In den **Figuren 2a** bis **2c** ist das in der EP 0 877 913 beschriebene Weglängenvariationselement **23** dargestellt, wobei hier die Zylindermantelflächen **49a** bis **49d** ausgehend von den Ecken **47a** bis **47d** nur eine teilweise annähernd 100 %-ige Beschichtung **50a** bis **50d** haben. Bei in **Figur 10** dargestellten Variante eines Weglängenvariationselements **147** mit ebenfalls einem quadratischen Querschnitt sind zwei benachbarte Zylindermantelflächen **149a** und **149b** vollständig mit einer annähernd 100 % reflektierenden Beschichtung versehen und die anderen beiden Zylindermantelfläche **151a** und **151b** ohne reflektierende Beschichtung, vorzugsweise mit einer antireflektierenden Beschichtung versehen. Das Weglängenvariationselement **147** rotiert ebenfalls um eine Rotationsachse **152.** Bei einem Brechungsindex des Materials des Weglängenvariationselements **147** von n = 1,5 ist eine Drehung von 0 ° bis 58 ° möglich, ohne dass die Weglängenvariation abbricht. Hierdurch ist die maximal mögliche Scantiefe für eine Zylindermantelfläche gegenüber dem in den **Figuren 2a** bis **2c** dargestellten Weglängenvariationselement **23** in jeweils einem Referenzarm vergrössert. (Beim Weglängenvariationselement **23** ist lediglich eine maximale Drehbewegung um 44 ° möglich.) Nachteilig bei einer die gesamte Seitenlänge bedeckenden Reflexionsbeschichtung (Weglängenvariationselements **147**) im Vergleich zu einer nur teilweisen Reflexionsbeschichtung (Weglängenvariationselements **23**) ist, dass pro Referenzarm nur zweimal statt viermal pro Umdrehung gemessen werden kann. Damit sich bei einer vollständigen Seitenbeschichtung keine störenden zeitlichen Überlappungen während der Scanzeit der zwei Referenzarme ergeben, muss der Winkel δ**ₓ** zwischen den zwei auf das Weglängenvariationselement **147** treffenden Strahlen **154a** und **154b** der beiden Referenzarme nicht 45 °, sondern mindestens 58 ° betragen.

In **Figur 11** ist eine Vorrichtung zur Ermittlung von wenigstens einem geometrischen Wert an transparenten oder diffusiven Gegenständen mit einem optischen Zeitraum-Kohärenztomographen TDOCT **155** und einem optischen Spektralraum-Kohärenztomographen SDOCT **156** dargestellt. Die Komponenten des TDOCTs **155** sind mit einer punktierten Linie umzogen und diejenigen des SDOCTs **156** sind mit einer strichpunktierten Linie umzogen. Der TDOCT **155** enthält im Wesentlichen die in **Figur 1** dargestellten Komponenten. Durch eine Verbindung von TDOCT **155** mit einem SDOCT **156** können nun die Vorteile dieser beiden Tomographen miteinander vereint werden. Neben einer Ermittlung einer Dicke, eines Abstands und/oder einer Länge kann nun auch eine Topographie schnell und genau ermittelt werden. Diese vereinte Messung ist zwar auch mit der oben beschriebenen, in **Figur 1** dargestellten Vorrichtung mit einem entsprechend ausgebildeten, oben beschriebenen Messkopf **20** möglich, jedoch ergibt die Verwendung eines SDOCT **156** anstelle der oben beschriebenen LED-Anordnungen Tomographiedaten, welche exakter und schneller ermittelbar sind, wobei zudem im Inneren des Gegenstands Topographien ermittelt werden können.

Ein SDOCT **156** alleine ist z. B. in der Veröffentlichung von R.A.Leitgeb, W. Drexler, A. Untergrube, B. Hermann, T. Bajraszewski, T. Ie, A. Stingl, A.F. Fercher, "Ultrahigh Resolution Fourier Domain Optical Coherence Tomography, Optics Express, Vol. 12, No. 10, 17. Mai 2004, S. 2156 - 2165, beschrieben und wird dort nicht zur Ermittlung einer Topographie, sondern zur Ermittlung von Schichtdicken verwendet.

Der SDOCT **156** misst nun bei einem beispielsweisen Einsatz am Auge entweder das vordere Augensegment oder das hintere Augensegment aus, weil die maximal mögliche Scantiefe im Gegensatz zum TDOCT **155,** beim SDOCT**156** typischerweise auf einige Millimeter beschränkt ist. Ein Grund für diese Beschränkung ist in der Anzahl zur Verfügung stehender Kamerapixel **157,** die in einer Kamerazeile **159** vorhanden sind, zu suchen; jede Zeile **159** hat typischerweise 1000 Pixel. Das TDOCT **155** ist im Gegensatz zum SDOCT **156** in der Lage, Längen von typischerweise 30 mm bis 40 mm zu messen. Die Messsensitivität und die Scangeschwindigkeit des SDOCTs **156** ist jedoch um einige Faktoren grösser als diejenige des TDOCTs **155.** Wegen der höheren Sensitivität und der höheren Geschwindigkeit wird mit dem SDOCT **156** im Messgegenstand eine Fläche (zweidimensional) bzw. ein Volumen (dreidimensional) mit einer Tiefe von typischerweise 3 mm gescannt, je nachdem wie ein unten beschriebener Spiegel **189** den Messstrahl **186a** in eine oder zwei Richtungen ablenkt. Das TDOCT **155** misst im Gegensatz zum SDOCT **156** im Messgegenstand nur in einer Dimension.

Der in **Figur 11** dargestellte TDOCTs **155** weist im Wesentlichen die bereits in **Figur 1** gezeigten Komponenten auf. Sofern die Komponenten mit denjenigen in der **Figur 1** identisch sind, haben sie gleiche Bezugszeichen. Analog zu **Figur 1** sind somit eine Strahlungsquelle **1,** ein Strahlungsabschwächer **5,** ein Polarisationskontroller **7** sowie analog ausgebildete Referenzarme **11** und **12** mit einem Weglängenvariationselement **23** vorhanden, wobei das Weglängenvariationselement **23** auch als Weglängenvariationselement **103** bzw. **147** ausgebildet sein kann. Ferner hat der TDOCT **155,** wie in **Figur 1** dargestellt, die beiden Detektoren **15** und **16** und die Detektionselektronik **17.** Der in **Figur** 1 vorhandene Messkopf **20** und der Monomodefaserkoppler **9** sind jedoch anders ausgebildet und sind unten beschrieben.

Die in **Figur 11** gezeigte Anordnung hat neben den oben bereits angeführten Komponenten einen 4 x 4 - Monomodefaserkoppler **160,** der auf seiner rechten Seite in **Figur 11** mit den Strahlungsleitern **3c, 10b, 10a** und einem Strahlungsleiter **161** verbunden ist, der zu einem Spektrometer **163** führt.

Das Spektrometer **163** hat eine Linse **164,** welche die aus dem Strahlungsleiter **161** austretende Strahlung **165** kollimiert. Die kollimierte Strahlung **167** wird auf ein Gitter **169** geführt. Das Gitter **169** reflektiert jede Wellenlänge, die in dem auf das Gitter **169** auftreffenden Strahlung **167** enthalten sind in eine andere Richtung. Diese nach den Wellenlängenanteilen zerlegte Strahlung **170** wird mittels einer Fokussierlinie **171** auf die die Kamerapixel **157** aufweisende Kamerazeile **159** fokussiert. Jedes Pixel **157** empfängt somit immer nur einen ganz bestimmten Wellenlängenbereich. Mit der Ermittlung der Intensitäten der von den Kamerapixeln detektierten Wellenlängenbereiche und mit Hilfe einer mathematischen Umformung (Fouriertransformation) in einer Auswerteeinheit **173** können die Positionen der verschiedenen im Gegenstand **19** vorkommenden Reflexionen und damit eine Topographie bzw. ein Meridian ermittelt werden.

Auf der linken Seite in **Figur 11** ist der 4 x 4 - Monomodefaserkoppler **160** mit den beiden Referenzarmen **11** und **12,** einem Messarm **175,** der sich vom Messarm **13** unterscheidet und einem weiteren Referenzarm **176** verbunden. Der Referenzarm **176** ist über eine Monomodefaser **177a** mit dem Koppler **160** verbunden. Das andere Ende der Monomodefaser **177a** ist mit einem optionalen, z. B. piezo-elektrisch arbeitenden Phasenmodulator **179** und dieser über eine Monomodefaser **177b** mit einem optinalen Polarisationskontroller **180** verbunden. Ausgehend von den Polarisationskontroller **180** gelangt die Strahlung auf einen Spiegel **181,** von dem die Strahlung wieder über die vorgängig angeführten Komponenten zum Koppler **160** zurück reflektiert wird.

Der mit dem Koppler **160** verbundene Messarm **175** weist den in **Figur 1** dargestellten Polarisationskontroller **18** und zur Erzeugung einer Freiraumstrahlung die Ferrule **57** auf. Mit einer Linse und einem analog zu den optischen Elementen **61, 63** oder **130** ausgebildeten optischen Element **184** ist eine Fokuspunktverstellung synchronisiert zur Weglängenmessung in den Referenzarmen **11** und **12,** wie oben beschrieben, vornehmbar. Zwischen der Linse **183** und dem optischen Element **184** ist ein Teilerspiegel **185** angeordnet. Die mit dem Teilerspiegel **185** ausgekoppelte Strahlung **186a** wird über einen Umlenkspiegel **187** auf einen Schwenkspiegel **189** und dann auf einen weiteren Teilerspiegel **190** geführt. Auf dem Teilerspiegel **190** erfolgt eine Vereinigung der vom Tellerspiegel **185** nicht ausgekoppelten Strahlung **186b** mit der über die Spiegel **187** und **189** umgelenkten Strahlung **186a.** Durch die Bewegung des Schwenkspiegels **189** wird der Teilstahl **186a,** der Teil des optischen Spektralraumtomographen **156** ist, seitlich über das zu messende beispielsweise vordere oder hintere Augensegment abgelenkt.

Im Faserkoppler **160** wird die vom Gegenstand 19 rückreflektierte Strahlung mit der vom Referenzarm **176** reflektierten Strahlung überlagert. Vom Faserkoppler **160** gehen dann 25 % der Strahlung in den Strahlungsleiter **161.**

Der TDOCT **155** enthält somit als Hauptkomponenten das Weglängenvariationselement **23,** die beiden Referenzarme **11** und **12,** den 4 x 4 - Monomodefaserkoppler **160,** die Strahlungsquelle **1,** den Strahlungsabschwächer **5,** den Polarisationskontroller **7,** die beiden Detektoren **15** und **16,** die Detektionselektronik **17,** die Auswerteeinheit **173,** den Polarisationskontroller **18,** die Ferrule **57,** die Linse 183, die optische Einheit **184** und der Gegenstand (Auge) **19.**

Der SDOCT **156** enthält die Strahlungsquelle **1,** den Strahlungsabschwächer **5,** den Polarisationskontroller **7,** den 4 x 4 - Monomodefaserkoppler **160,** das Spektrometer **163,** den Referenzarm **176,** den Polarisationskontroller **18,** die Ferrule **57,** die Linse **183,** den Teilerspiegel **185,** den Umlenkspiegel **187,** den Schwenkspiegel **189,** den Teilerspiegel **190** und den Gegenstand **19.** In einer vorteilhaften Ausgestaltung wird man zwischen dem Schwenkspiegel **189** und dem Umlenkspiegel **187** bzw. dem Teilerspiegel **185** eine (in **Figur 11** nicht dargestellte) Linse anordnen, mit der die Strahlung auf das Gebiet im Gegenstand **19** fokussierbar ist, dessen "innere" Topographie ermittelt werden soll, wobei selbstverständlich auch eine "äussere" Topographie ermittelbar ist. Diese Linse wird man jedoch nicht zwischen dem Schwenkspiegel **189** und dem Teilerspiegel **190** anordnen, da dann der Strahl infolge der Schwenkspiegelablenkung auf der Linsenoberfläche wandern würde.

Die in **Figur 11** dargestellte Anordnung kann zusätzlich die in **Figur 3** gezeigten Komponenten zur Ermittlung einer Topographie enthalten. Um **Figur 11** nicht zu überladen sind lediglich der wellenlängenselektive Spiegel 71, die Linseneinheit **72,** das Sperrfilter **75,** und der Kamerachip **73** eingezeichnet. Die LED-Anordnungen sind selbstverständlich auch vorhanden, aber nicht eingezeichnet. Mit der Anordnung analog **Figur 3** kann die Topographie von freien Oberflächen bestimmt werden, während mit den SDOCT **156** neben freien Oberflächen auch in der Gegenstandstiefe liegende Topographien ermittelt werden können.

Mit den oben beschriebenen Vorrichtungen ist eine Messung mehrerer Augenstrukturen in einem einzigen Positioniervorgang möglich. Es kann somit neben den jeweiligen Topographien aussen oder innen liegender Flächen beispielsweise beim Auge die Dicke und die innere Struktur der Hornhaut, der Vorderkammer, der Augenlinse, des Glaskörpers ermittelt werden. Zudem kann ferner die Linsenrinde und eine Linsentrübung, die Augenlänge, die innere Struktur der Netzhaut, die Position und die Dicke von phaken und pseudophaken intraokularen Linsen, die Position und die Dicke von Kontaktlinsen, der Krümmungsradius der Hornhaut mittels eines einzigen am Patientenauge vorgenommenen Positioniervorgang der Vorrichtung ermittelt sowie der Kammerwinkel ausgemessen werden.

## Patentansprüche

1. Verfahren zum Messen wenigstens eines geometrischen Werts, wie beispielsweise einer Dicke, eines Abstands oder einer Länge, an einem transparenten oder diffusiven Gegenstand **(19)** mittels optischer Zeitraum-Kohärenztomographie (**t**ime domain optical coherence tomographie TDOCT) **(155),** wobei
a) mit einer Strahlungsquelle **(1)** eine Strahlung kurzer Kohärenzlänge erzeugt wird,
b) die Strahlung in einen Messarm **(13)** und in wenigstens zwei Referenzarme **(11, 12)** eines Michelson-Interferometers aufgeteilt wird,
c) in den wenigstens zwei Referenzarmen **(11, 12)** jeweils Referenzarmstrahlen **(33a, 35a)** gebildet und auf ein rotierendes Weglängenvariationselement **(23; 103; 147)** geführt werden,
d) in Abhängigkeit einer Rotation **(45, 118)** des Weglängenvariationselements **(23; 103; 147)** eine drehwinkelabhängige Laufzeitänderung der Referenzarmstrahlen erzeugt wird,
e) der auszumessende Gegenstand **(19)** in den Messarm **(13)** gebracht wird,
f) optische Grundlaufzeiten der Referenzarme **(11, 12)** derart gewählt werden, dass eine optische Laufzeitdifferenz der Strahlungen in den wenigstens zwei Referenzarmen **(11, 12)** annähernd einer optischen Laufzeitdifferenz des zu messenden geometrischen Werts entspricht,
g) am Gegenstand **(19)** und in den Referenzarmen **(11, 12)** reflektierte Strahlung interferenzmässig überlagert und sich jeweils ergebende Strahlungsinterferenzen detektiert werden,
**dadurch gekennzeichnet, dass**
h) die wenigsten zwei Referenzarmstrahlen **(33a, 35a)** der Referenzarme **(11, 12)** unter einem gegenseitigen räumlichen Versetzungswinkel **(**δ**_{W},** δ**_{S})** auf ein und dasselbe rotierende Weglängenvariationselement **(23; 103; 147)** geführt werden,
i) derart dass die Weglängenvariation zuerst im ersten Referenzarm und dann im zweiten Referenzarm wirkt, indem den optischen Grundlaufzeiten in den Referenzarmen **(11, 12)** aufeinander folgend eine durch das Weglängenvariationselement **(23; 103)** hervorgerufene Laufzeitänderung aufgeprägt wird, wodurch die den geometrischen Wert definierenden Strahlungsinterferenzen zeitlich voneinander getrennt auftreten.

2. Verfahren nach Anspruch 1, **dadurch gekennzeichnet, dass** die Strahlung **(22)** im Messarm **(13)** jeweils auf eine erste und eine zweite, den auszumessenden geometrischen Wert definierende Schichtbegrenzung am Gegenstand synchron bzw. periodisch mit der Rotation fokussiert wird.

3. Verfahren nach Anspruch 1 oder 2, **dadurch gekennzeichnet, dass** das Weglängenvariationselement **(23)** ein im Querschnitt regelmässiges Vieleck bildet, wobei die Referenzstrahlen **(33b, 33c, 33d; 35b, 35c, 35d)** innerhalb des Elements **(23),** an wenigstens zwei der Mantelflächen **(49a-d)** des Vielecks reflektiert werden.

4. Verfahren nach einem der Ansprüche 1 bis 3, **dadurch gekennzeichnet, dass** vor dem auszumessenden Gegenstand **(19)** eine vorgegebene optische Struktur **(70; 79; 87; 91)** angeordnet wird und die Reflexionen dieser Struktur **(70; 79; 87; 91)** auf dem Gegenstand **(19)** abgebildet und die Abbildung mit der vorgegebenen Struktur **(70; 79; 87; 91)** zur Ermittlung einer Oberflächentopografie des Gegenstands **(19)** als weiteren geometrischen Wert verglichen werden, um neben den Dickenwerten der Gegenstandsschichten ein Oberflächenprofil bzw. ein Schichtenprofil zu ermitteln, und vorzugsweise der Strahl **(22)** des Messarms **(13)** mit einem wellenlängen-selektiven Spiegel **(71)** reflektierend zum Gegenstand **(19)** umgelenkt wird, wobei die von der vorgegebenen optischen Struktur **(70; 79; 87; 91)** ausgehende weitere Strahlung eine andere Wellenlänge als der Messarmstrahl hat, der Spiegel **(71)** die weitere Strahlung transmittiert und ein auf oder Gegenstandsoberfläche durch die weitere Strahlung erzeugtes Muster in rückwärtiger Verlängerung der Achse des auf den Gegenstand treffenden Messarmstrahls **(22)** auf der anderen Seite des Spiegels **(71)** abgebildet und detektiert wird, und insbesondere die optische Struktur **(70; 79; 87; 91)** mit mehreren LEDs **(89a,b; 90; 97n,m)** erzeugt wird, wobei vorzugsweise die Strahlungen der LEDs **(97n,m)** auf eine Oberfläche des Gegenstands **(19)** fokussiert werden.

5. Verfahren nach einem der Ansprüche 1 bis 4, **dadurch gekennzeichnet, dass** neben der optischen Zeitraum-Kohärenztomographie **(155)** eine optische Spektralraum-Kohärenztomographie **(156)** mit ein und derselben Strahlungsquelle verwendet wird, um neben der Ermittlung des geometrischen Werts zusätzlich eine Topographie zu ermitteln.

6. Vorrichtung zum Messen wenigstens eines geometrischen Werts, wie beispielsweise einer Dicke, eines Abstands oder einer Länge, eines transparenten oder diffusiven Gegenstands **(19),** mit einem optischen Zeitraum-Kohärenztomographen **(155)**
a) mit einem eine Strahlungsquelle **(1)** kurzer Kohärenzlänge aufweisenden Michelson-Interferometer mit einem Messarm **(13),** in den der auszumessende Gegenstand **(19)** einbringbar ist,
b) sowie mit wenigstens zwei Referenzarmen **(11, 12)** mit zueinander unterschiedlichen optischen Grundweglängen entsprechend dem wenigstens einen auszumessenden geometrischen Wert,
c) wobei in den wenigstens zwei Referenzarmen **(11, 12)** jeweils Mittel zum Erzeugen von Referenzarmstrahlen (33a, 35a) vorgesehen sind und
**gekennzeichnet durch**
d) ein einziges, in beiden Referenzarmen **(11, 12)** wirkendes, um eine Elementachse (21, 105) rotierendes, Weglängenvariationselement **(23; 103; 147),**
e) wobei die wenigstens zwei Referenzarmstrahlen der wenigstens zwei Referenzarme **(11, 12)** unter einem gegenseitigen Versetzungswinkel **(**δ**_{W};** δ**_{S};** δ**ₓ)** auf das Wegvariationselement **(23; 103; 147)** geführt sind, derart dass die Weglängenvariation zuerst im ersten Referenzarm und dann im zweiten Referenzarm wirkt, indem aufeinander folgend eine Weglängenvariation den optischen Grundweglängen aufprägbar ist.

7. Vorrichtung nach Anspruch 6, **dadurch gekennzeichnet, dass** das Wegvariationselement (23; 103; 147) mehrere Mantelflächen (49a-d; 109a-109l) hat, die Elementachse (21; 105) des Wegvariationselements (23; 103; 147) parallel zu den Mantelflächen (49a-d; 109a-1091) und senkrecht zu den Achsen der freien Referenzarmstrahlen (33a, 35a; 111 a, 121) angeordnet ist.

8. Vorrichtung nach Anspruch 6 oder 7, **dadurch gekennzeichnet, dass** das Weglängenvariationselement (23) als ein im Querschnitt regelmässiges Vieleck ausgebildet ist, wobei die Referenzstrahlen (33b, 33c, 33d; 35b, 35c, 35d) innerhalb des Elements (23), an wenigstens zwei der Mantelflächen (49a-d) des Vielecks reflektiert werden, und bevorzugt die Mantelflächen (49a-d) wenigstens eine Teilfläche (50a-d) aufweisen, welche annähernd 100% reflektierend für die Referenzarmstrahlen (33b, 33c, 33d; 35b, 35c, 35d) wirkt, und jede Teilfläche (50a-d) beginnend an jeder Mantelflächenecke (47a-d) für nur denjenigen innerhalb des Elements (23) reflektierbaren Strahlabschnitt (33b, 33c; 35b, 35c), der mit dem dazu gehörenden einfallenden Strahlabschnitt (33c, 33d; 35c, 35d) einen spitzen Winkel (β) bildet, annähernd 100%-ig reflektierend beschichtet ist, um eine möglichst verlustgeminderte Strahlführung zu erreichen.

9. Vorrichtung nach einem der Ansprüche 6 bis 8, **gekennzeichnet durch** eine im Messarm (13) des Michelson-Interferometers angeordnete, auf den freien Messarmstrahl (22) wirkende Fokussiereinheit (58), deren Fokusort auf wenigstens zwei unterschiedliche Messorte alternierend fokussierbar ist, wobei die Fokussierung auf die jeweiligen Messorte synchronisiert mit der Rotation des Wegvariationselements (23) vornehmbar ist, und vorzugsweise die Fokussiereinheit (58) eine Linseneinheit (59) hat, wobei im Messstrahlengang vor oder hinter der Linseneinheit (59), in einem im Wesentlichen von einer parallelen Strahlkonfiguartion abweichenden Strahllängsbereich, eine über den Messstrahlquerschnitt bewegbare optische Einheit (61, 63; 130) angeordnet ist, mit der eine unterschiedliche Strahlkonfigurationen des Messstrahls (22) zur Erzeugung einer Fokuspunktverschiebung synchronisiert mit der Rotation des Wegvariationselements (23) einstellbar ist, und insbesondere die optische Einheit (61, 63; 130) wenigstens einen in den Messstrahl (22) einbringbaren, transparenten, planparallel ausgebildeten Bereich mit einer vorgegebenen optischen Länge für den Messstrahl (22) aufweist, welche mit der Fokussiereinheit (59) eine Fokuspunktverschiebung von einer Dickenbegrenzungsfläche zur anderen ergibt, wobei der wenigstens eine Bereich derart angeordnet ist, dass er während einer der aktiven, alternierenden Messzeiten des jeweiligen Referenzarmes (11, 12) im Messstrahl (22) befindlich ist, und vorzugsweise die optische Einheit als rotierende Kreisscheibe (63; 130) mit wenigstens einem transparenten Festkörpersegment (64; 135, 137) ausgebildet ist.

10. Vorrichtung nach einem der Ansprüche 6 bis 9, **gekennzeichnet durch** mehrere Beleuchtungsstrahlungsquellen (70), welche vor dem Gegenstand (19), insbesondere einem menschlichen Auge als Gegenstand (19), angeordnet sind, und mit einer optischen Aufnahmeeinheit (73), mit der Reflexionsorte der Beleuchtungsstrahlungsquellen (80a, 80b; 89a, 89b; 97n,m) auf der Gegenstandsoberfläche (69) abbildbar sind, und einer Auswerteeinheit (74), mit der die gegenseitigen Abstände der abgebildeten Reflexionsorte ins Verhältnis zu den Abständen der Strahlungsquellen (80a, 80b; 89a, 89b; 97n,m) zur Ermittlung einer Oberflächenkrümmung der Gegenstandsoberfläche (69) als weiterer geometrischen Wert setzbar sind, und vorzugsweise vor den Strahlungsquellen (97n,m) angeordnete Fokussierelemente (99), mit denen die Strahlung der Beleuchtungsstrahlungsquellen (97n,m) auf unterschiedliche Bereiche im transparenten Gegenstand (19) fokussierbar ist, wobei beim menschlichen Auge als Gegenstand (19) mit den Fokussierelementen eine Fokussierung auf die Hornhautvorderfläche und/oder auf die Homhautrückfläche und/oder auf die Linsenvorderfläche und/oder auf die Linsenrückfläche vornehmbar ist, wobei insbesondere die Beleuchtungsstrahlungsquellen LEDs (80a, 80b; 89a, 89b; 97n,m) sind, welche vorzugsweise eine Strahlung mit einer anderen Wellenlänge emittieren als die Strahlungsquelle (1) des Michelson-Interfermeters, und vorzugsweise die Fokussierelemente Zylinderlinsen (99a-h) sind, wobei zur Vermeidung einer Vielzahl von Zylinderlinsen ein bzw. mehrere über den Strahlungsquellen rotierenden Zylinderlinsen anbringbar sind.

11. Vorrichtung nach einem der Ansprüche 6 bis 10, **gekennzeichnet durch** Strahlungsleiter (25a, 25b, 26a, 26b, 14a, 14b), mit denen die Strahlung der Referenzarme (11, 12) und diejenige des Messarms (13) führbar ist und an den Strahlungsleiterenden angeordnete Aus- bzw. Einkoppelelemente, mit denen unmittelbar vor bzw. nach optischen Komponenten (23; 58, 70, 71) bzw. vor dem Messobjekt (19) die im betreffenden Strahlungsleiter geführte bzw. zu führende Strahlung als Freiraumstrahl (22, 33a, 33d, 35a, 35d) aus- bzw. einkoppelbar ist.

12. Vorrichtung nach Anspruch 10, **gekennzeichnet durch** sich voneinander unterscheidende Wellenlängen der kurzkohärenten Strahlungsquelle (1) und wenigstens einer ersten Anzahl von Beleuchtungsstrahlungsquellen (80a, 80b; 89a, 89b; 97n,m), **durch** einen wellenlängen-selektiven Spiegel (71) im Messarm (13), mit dem der Strahl (22) der kurzkohärenten Strahlungsquelle (1) auf den Gegenstand (19) reflektierbar und die Strahlung der Beleuchtungsstrahlungsquellen (80a, 80b; 89a, 89b; 97n,m) transmittierbar sind, und **durch** eine Abbildungseinheit (72) für die vom Gegenstand (19, 69) reflektierbaren Strahlung der Beleuchtungsstrahlungsquellen (80a, 80b; 89a, 89b; 97n,m), wobei vor dem Gegenstand (19) die optische Achse des Messarms (13) mit der optischen Achse der Abbildungseinheit zusammenfallen.

13. Vorrichtung nach den Ansprüchen 9 und 10, **gekennzeichnet durch**, einen 3 x 3 - Monomodefaserkoppler (9), wobei die Messstrahlung ausgehend von dem 3 x 3 - Monomodefaserkoppler (9) in einem ersten Strahlungsleiter (14a) führbar ist, und der Monomodefaserkoppler (9) mit der Michelson-Strahlungsquelle (1) über einen zweiten Strahlungsleiter (3c), mit zwei Referenzarmen (11, 12) über einen dritten und vierten Strahlungsleiter (25a, 26a) sowie zu einen ersten und zweiten Interferenzdetektor (15, 16) über einen fünften und sechsten Strahlungsleiter (10a, 10b) verbunden ist, und das andere Ende (14b) des ersten Strahlungsleiters (14a) unmittelbar vor die Fokussiereinheit (58) geführt ist, nach der Fokussiereinheit (58) ein Wellenlängen selektiver Spiegel (71) angeordnet ist, mit dem die Messstrahlung (22) auf den auszumessenden Gegenstand (19) lenkbar ist und die vom Gegenstand (19) reflektierte Messstrahlung (22) zurück in die Fokussiereinheit (58) und in den ersten Strahlungsleiter (14a, 14b) führbar ist, und die Beleuchtungsstrahlungsquellen (80a, 80b; 89a, 89b; 97n,m) zwischen dem Spiegel (71) und dem Gegenstand (19), vorzugsweise in einer Ebene senkrecht zur Achse des Messstrahls (22) in unmittelbarer Nähe des Spiegels (71), angeordnet sind und in rückwärtiger Verlängerung des Messstrahls (22) auf der dem Gegenstand (19) abgewandten Seite des Spiegels (71) ein Abbildungssystem (72) vor einer optischen Auswerteeinheit (73) für die auf bzw. im Gegenstand (19) reflektierten Beleuchtungsstrahlenquellen (80a, 80b; 89a, 89b; 97n,m) angeordnet ist.

14. Vorrichtung nach einem der Ansprüche 6 bis 9, **dadurch gekennzeichnet, dass** neben dem optischen Zeitraum-Kohärenztomographen (155) ein optischer Spektralraum-Kohärenztomograph (156) vorhanden ist, wobei beide Tomographen (155, 156) mit ein und derselben Strahlungsquelle (1) arbeiten.

15. Vorrichtung nach Anspruch 14, **gekennzeichnet durch** ein Spektrometer (163) und einen 4 x 4 - Monomodefaserkoppler (160), wobei die Messstrahlung für den optischen Zeitraum-Kohärenztomographen (155) und den optischen Spektralraum-Kohärenztomographen (156) in einem ersten Strahlungsleiter (14a, 14b) führbar ist, und der Monomodefaserkoppler (160) mit der Strahlungsquelle (1) über einen zweiten Strahlungsleiter (3a-c), mit den zwei Referenzarmen (11, 12), welche ein gemeinsames Weglängenvariationselement (23) aufweisen, über einen dritten und einen vierten Strahlungsleiter (25a, 26a), zu einem ersten und zweiten Interferenzdetektor (15, 16) über einen fünften und sechsten Strahlungsleiter (10a, 10b), mit einem dritten Referenzarm (176) über einen siebten Strahlungsleiter (177a) und mit dem Spektrometer (163) über einen achten Strahlungsleiter (161) verbunden ist.

## Claims

1. Method for measurement of at least one geometric value such as, for example, a thickness, a distance or a length, on a transparent or diffusive object (**19**) by means of **T**ime **D**omain **O**ptical **C**oherence **T**omography (**TDOCT**) (**155**), in which case:
a) a radiation of short coherence length is generated with a radiation source (**1**),
b) the radiation is split into a measurement arm (**13**) and into at least two reference arms (**11, 12**) of a Michelson interferometer,
c) reference arm beams (**33a**, **35a**) are respectively formed in the at least two reference arms (**11, 12**) and guided to a rotating path length variation element (**23; 103; 147**),
d) a change in delay time of the reference arm beams that is a function of a rotation (**45, 118**) of the path length variation element (**23; 103; 147**) is produced,
e) the object (**19**) to be measured is brought into the measurement arm (**13**),
f) fundamental optical delay times of the reference arms (**11, 12**) are selected in such a way that an optical delay time difference of the beams in the at least two reference arms (**11, 12**) corresponds approximately to an optical delay time difference of the geometric value to be measured, and
g) radiation reflected at the object (**19**) and in the reference arms (**11, 12**) is superposed together with interference, and respectively resulting radiation interferences are detected,
**characterized in that**
h) the at least two reference arm beams (**33a, 35a**) of the reference arms (**11, 12**) are guided to one and the same rotating path length variation element (**23; 103; 147**) at a mutual spatial offset angle (δ**_{w},** δ**ₛ**),
i) in such a way that the path length variation initially acts in the first reference arm and then in the second reference arm by virtue of the fact that a change in delay time caused by the path length variation element (**23; 103**) is successively impressed on the fundamental optical delay times in the reference arms (**11, 12**), as a result of which the radiation interferences defining the geometric value occur with temporal separation from one another.

2. Method according to claim 1, **characterized in that** the radiation (**22**) in the measurement arm (**13**) in each case is focused on a first and a second layer boundary, defining the geometric value to be measured, on the object synchronously or periodically with the rotation.

3. Method according to claim 1 or 2, **characterized in that** the path length variation element (**23**) forms a regular polygon in cross section, the reference beams (**33b, 33c, 33d; 35b, 35c, 35d**) being reflected inside the element (**23**) at at least two of the lateral surfaces (**49a-d**) of the polygon.

4. Method according to one of claims 1 to 3, **characterized in that** a prescribed optical structure (**70; 79; 87; 91**) is arranged upstream of the object (**19**) to be measured, and the reflection of this structure (**70; 79; 87; 91**) is imaged on the object (**19**), and the image is compared with the prescribed structure (**70; 79; 87; 91**) for the purpose of determining a surface topography of the object (**19**) as further geometric value in order to determine a surface profile or a layer profile in addition to the thickness values of the object layers, and the beam (**22**) of the measurement arm (**13**) is preferably deflected in a reflecting fashion to the object (**19**) with the aid of a wavelength-selective mirror (**71**), the further radiation emanating from the prescribed optical structure (**70; 79; 87; 91**) having a different wavelength than the measurement arm beam, the mirror (**71**) transmitting the further radiation, and a pattern produced on the object surface by the further radiation being imaged on the other side of the mirror (**71**) in a backward extension of the axis of the measurement arm beam (**22**) striking the object, and being detected, and, in particular, the optical structure (**70; 79; 87; 91**) being produced with a number of LEDs (**89a,b; 90; 97n,m**), the radiations of the LEDs (**97n,m**) preferably being focused onto a surface of the object (**19**).

5. Method according to one of claims 1 to 4, **characterized in that**, in addition to the time domain optical coherence tomography (**155**), use is made of a spectral domain optical coherence tomography (**156**) with one and the same radiation source in order additionally to determine a topography in addition to the determination of the geometric value.

6. Apparatus for measurement of at least one geometric value such as, for example, a thickness, a distance or a length, of a transparent or diffusive object (**19**) with the aid of a time domain optical coherence tomograph (**155**),
a) comprising a Michelson interferometer, having a radiation source (**1**) of short coherence length, with a measurement arm (**13**) into which the object (**19**) to be measured can be brought,
b) as well as comprising at least two reference arms (**11, 12**) with mutually differing fundamental optical path lengths corresponding to the at least one geometric value to be measured,
c) means respectively being provided in the at least two reference arms (**11, 12**) for the purpose of generating reference arm beams (**33a, 35a**), and
**characterized by**
d) a single path length variation element (**23; 103; 147**) acting in both reference arms (**11, 12**) and rotating about an element axis (**21, 105**),
e) the at least two reference arm beams of the at least two reference arms (**11, 12**) being guided onto the path variation element (**23; 103; 147**) at a mutual offset angle (δ**_{w}**; δ**ₛ**; δ**ₓ**) in such a way that the path length variation initially acts in the first reference arm and then in the second reference arm by virtue of the fact that a path length variation can be successively impressed on the fundamental optical path lengths.

7. Apparatus according to claim 6, **characterized in that** the path variation element (23; 103; 147) has a number of lateral surfaces (49a-d; 109a-1091), while the element axis (21; 105) of the path variation element (23; 103; 147) is arranged parallel to the lateral surfaces (49a-d; 109a-1091) and perpendicular to the axes of the free reference arm beams (33a, 35a; 111a, 121).

8. Apparatus according to claim 6 or 7, **characterized in that** the path length variation element (23) is designed as a regular polygon in cross section, the reference beams (33b, 33c, 33d; 35b, 35c, 35d) being reflected inside the element (23) at at least two of the lateral surfaces (49a-d) of the polygon, and the lateral surfaces (49a-d) preferably having at least one partial surface (50a-d) that acts to reflect approximately 100% of the reference arm beams (33b, 33c, 33d; 35b, 35c, 35d), and each partial surface (50a-d), beginning at each lateral surface corner (47a-d), being coated to reflect approximately 100% for only that beam section (33b, 33c; 35b, 35c) that can be reflected inside the element (23) and forms an acute angle (β) with the incident beam section (33c, 33d; 35c, 35d) belonging thereto, in order to achieve a beam guidance with the greatest possible loss reduction.

9. Apparatus according to one of claims 6 to 8, **characterized by** a focusing unit (58) that is arranged in the measurement arm (13) of the Michelson interferometer and acts on the free measurement arm beam (22) and whose focal point can be alternatingly focused on at least two different measuring points, it being possible to undertake the focusing onto the respective measuring points in a fashion synchronized with the rotation of the path variation element (23), and the focusing unit (58) preferably having a lens unit (59), there being arranged in the measuring beam path upstream or downstream of the lens unit (59), in a beam longitudinal region deviating substantially from a parallel beam configuration, an optical unit (61, 63; 130) that can be moved over the measuring beam cross section and with the aid of which it is possible to set a different beam configuration of the measuring beam (22) for the purpose of producing a focal point displacement in a fashion synchronized with the rotation of the path variation element (23) and, in particular, the optical unit (61, 63; 130) having at least one transparent region of plane parallel design that can be brought into the measuring beam (22) and has a prescribed optical length for the measurement beam (22) that produces with the aid of the focusing unit (59) a focal point displacement from one thickness limiting surface to the other, the at least one region being arranged in such a way that it can be located in the measuring beam (22) during one of the active, alternating measurement times of the respective reference arm (11, 12), and the optical unit preferably being designed as a rotating circular disk (63; 130) with at least one transparent solid segment (64; 135, 137).

10. Apparatus according to one of claims 6 to 9, **characterized by** a number of illuminating radiation sources (70) that are arranged upstream of the object (19), in particular a human eye as object (19), and can be imaged on the object surface (69) with the reflection points of the illuminating radiation sources (80a, 80b; 89a, 89b; 97n,m) with the aid of an optical recording unit (73), and an evaluation unit (74) with the aid of which the mutual spacings of the imaged reflection points can be set in relationship to the spacings of the radiation sources (80a, 80b; 89a, 89b; 97n,m) for the purpose of determining a surface curvature of the object surface (69) as further geometric value, and preferably focusing elements (99) that are arranged upstream of the radiation sources (97n,m) and with the aid of which the radiation of the illuminating radiation sources (97n,m) can be focused onto different regions in the transparent object (19), it being possible in the case of the human eye as object (19) to use the focusing elements to focus onto the front corneal surface and/or onto the rear corneal surface and/or onto the lens front surface and/or onto the lens rear surface, the illuminating radiation sources being, in particular, LEDs (80a, 80b; 89a, 89b; 97n,m) that preferably emit a radiation of a different wavelength than the radiation source (1) of the Michelson interferometer, and the focusing elements being preferably cylindrical lenses (99ah), it being possible for the purpose of avoiding a multiplicity of cylindrical lenses to mount one or more rotating cylindrical lenses over the radiation sources.

11. Apparatus according to one of claims 6 to 10, **characterized by** radiation conductors (25a, 25b, 26a, 26b, 14a, 14b) with the aid of which the radiation of the reference arms (11, 12) and that of the measurement arm (13) can be guided, and outcoupling and incoupling elements, arranged at the ends of the radiation conductors, with the aid of which the radiation guided or to be guided in the relevant radiation conductor can be coupled out or coupled in as a free space beam (22, 33a, 33d, 35a, 35d) immediately upstream or downstream of optical components (23; 58, 70, 71) or upstream of the measurement object (19).

12. Apparatus according to claim 10, **characterized by** mutually different wavelengths of the short coherence radiation source (1) and at least of a first number of illuminating radiation sources (80a, 80b; 89a, 89b; 97n,m), by a wavelength-selective mirror (71) in the measurement arm (13) with the aid of which the beam (22) of the short coherence radiation source (1) can be reflected onto the object (19), and the radiation of the illumination radiation sources (80a, 80b; 89a, 89b; 97n,m) can be transmitted, and by an imaging unit (72) for the radiation, that can be reflected by the object (19, 69), of the illuminating radiation sources (80a, 80b; 89a, 89b; 97n,m), the optical axis of the measurement arm (13) coinciding with the optical axis of the imaging unit upstream of the object (19).

13. Apparatus according to claims 9 and 10, **characterized by** a 3 x 3 monomode fiber coupler (9), it being possible for the measurement radiation emanating from the 3 x 3 monomode fiber coupler (9) to be guided in a first radiation conductor (14a), and the monomode fiber coupler (9) being connected to the Michelson radiation source (1) via a second radiation conductor (3c), to two reference arms (11, 12) via a third and fourth radiation conductor (25a, 26a) and to a first and second interference detector (15, 16) via a fifth and sixth radiation conductor (10a, 10b), and the other end (14b) of the first radiation conductor (14a) being guided directly upstream of the focusing unit (58), there being arranged downstream of the focusing unit (58) a wavelength-selective mirror (71) with the aid of which the measurement radiation (22) can be directed onto the object (19) to be measured, and it being possible for the measurement radiation (22) reflected by the object (19) to be carried back into the focusing unit (58) and into the first radiation conductor (14a, 14b), and for illuminating radiation sources (80a, 80b; 89a, 89b; 97n,m) to be arranged between the mirror (71) and the object (19), preferably in a plane perpendicular to the axis of the measuring beam (22) in the immediate vicinity of the mirror (71), and an imaging system (72) being arranged, in a rearward extension of the measuring beam (22) on the side of the mirror (71) averted from the object (19), upstream of an optical evaluation unit (73) for the illuminating radiation sources (80a, 80b; 89a, 89b; 97n,m) reflected on or in the object (19).

14. Apparatus according to one of claims 6 to 9, **characterized in that** a spectral domain optical coherence tomograph (156) is present in addition to the time domain optical coherence tomograph (155), the two tomographs (155, 156) operating with the aid of one and the same radiation source (1).

15. Apparatus according to claim 14, **characterized by** a spectrometer (163) and a 4 x 4 monomode fiber coupler (160), it being possible for the measurement radiation for the time domain optical coherence tomograph (155) and the spectral domain optical coherence tomograph (156) to be guided in a first radiation conductor (14a, 14b), and the monomode fiber coupler (160) being connected to the radiation source (1) via a second radiation conductor (3a-c), to the two reference arms (11, 12), which have a common path length variation element (23), via a third and a fourth radiation conductor (25a, 26a), to a first and second interference detector (15, 16) via a fifth and sixth radiation conductor (10a, 10b), to a third reference arm (176) via a seventh radiation conductor (177a), and to the spectrometer (163) via an eighth radiation conductor (161).

## Revendications

1. Procédé de mesure d'au moins une grandeur géométrique, comme par exemple une épaisseur, une distance ou une longueur, sur un objet (19) transparent ou diffusant au moyen de la tomographie de cohérence optique dans le domaine temporel (time domain optical coherence tomography TDOCT) (155), dans lequel
a) un rayonnement de courte longueur de cohérence est généré au moyen d'une source de rayonnement (1),
b) le rayonnement est réparti dans un bras de mesure (13) et dans au moins deux bras de référence (11, 12) d'un interféromètre de Michelson,
c) dans les deux bras de référence (11, 12) au moins sont formés respectivement des rayons des bras de référence (33a, 35a) qui sont acheminés sur un élément rotatif de variation de la longueur du chemin (23 ; 103 ; 147),
d) une modification du temps de transit des rayons de référence est générée en fonction d'une rotation (45, 118) de l'élément de variation de la longueur du chemin (23 ; 103 ; 147),
e) l'objet (19) à mesurer est amené dans le bras de mesure (13),
f) les temps de transit optiques fondamentaux des bras de référence (11, 12) sont choisis de telle sorte qu'une différence des temps de transit optiques des rayons dans les deux bras de référence (11, 12) au moins, corresponde approximativement à une différence du temps de transit optique de la grandeur géométrique à mesurer,
g) le rayonnement réfléchi sur l'objet (19) et dans les bras de référence (11, 12) est superposé de manière à interférer et les interférences de rayonnement en résultant respectivement sont détectées,
**caractérisé en ce que**
h) les deux rayons des bras de référence (33a, 35a) au moins des bras de référence (11, 12) sont acheminés avec un angle de décalage spatial mutuel (δ_{w}, δₛ) sur un élément commun tournant de variation de la longueur du chemin (23 ; 103 ; 147),
i) de telle sorte que la variation de la longueur du chemin agisse d'abord dans le premier bras de référence et ensuite dans le deuxième bras de référence, du fait qu'une modification du temps de transit engendrée par l'élément de variation de la longueur du chemin (23 ;103) s'applique successivement aux temps de transit optiques fondamentaux dans les bras de référence (11, 12), ce qui entraine l'apparition successive séparée dans le temps des interférences des rayonnements définissant la grandeur géométrique.

2. Procédé selon la revendication 1, **caractérisé en ce que** le rayonnement (22) dans le bras de mesure (13) est focalisé de manière synchrone et/ou périodique avec la rotation, respectivement sur une première et une deuxième limite de couche définissant la grandeur géométrique à mesurer sur l'objet

3. Procédé selon la revendication 1 ou 2, **caractérisé en ce que** l'élément de variation de la longueur du chemin (23) forme un polygone régulier en coupe transversale, dans lequel les rayons de référence (33b, 33c, 33d; 35b, 35c, 35d) se réfléchissent à l'intérieur de l'élément (23) sur au moins deux des surfaces du revêtement extérieur (49a-d) du polygone.

4. Procédé selon une des revendications 1 à 3, **caractérisé en ce qu'**une structure optique définie (70 ; 79 ; 87 ; 91) est disposée devant l'objet (19) à mesurer et les réflexions de cette structure (70 ; 79 ; 87 ; 91) sont reproduites sur l'objet (19) et l'image est comparée avec la structure définie (70 ; 79; 87; 91) pour déterminer une topographie de la surface de l'objet (19) sous la forme d'une autre grandeur géométrique, pour déterminer en plus des valeurs d'épaisseur des couches de l'objet, un profil des surfaces et/ou un profil de couches, et de préférence pour dévier le rayon (22) du bras de mesure (13) par réflexion sur un miroir (71) sélecteur de longueurs d'ondes vers l'objet (19), dans lequel l'autre rayonnement partant de la structure optique définie (70 ; 79 ; 87 ; 91) a une longueur d'onde différente de celle du rayon du bras de mesure, le miroir (71) transmet l'autre rayonnement et reproduit et détecte un motif de l'autre côté du miroir (71), engendré à la surface de l'objet par l'autre rayonnement, dans le prolongement vers l'arrière de l'axe du rayon du bras de mesure (22) frappant l'objet, et engendre notamment la structure optique (70 ; 79 ; 87 ; 91) à plusieurs LEDs (89a,b ; 90 ; 97n,m), dans lequel les rayonnements des LEDs (97n,m) sont de préférence focalisés sur une surface de l'objet (19).

5. Procédé selon une des revendications 1 à 4, **caractérisé en ce qu'**en plus de la tomographie de cohérence optique dans le domaine temporel (155), on utilise une tomographie de cohérence optique dans le domaine spectral (156) avec une source de rayonnement commune pour déterminer de plus une topographie en plus de la détermination de la grandeur géométrique.

6. Dispositif de mesure d'au moins une grandeur géométrique, comme par exemple une épaisseur, une distance ou une longueur, sur un objet (19) transparent ou diffusant au moyen d'une tomographe de cohérence optique dans le domaine temporel (155),
a) avec un interféromètre de Michelson comportant une source de rayonnement (1) de courte longueur de cohérence avec un bras de mesure (13), dans lequel on peut placer l'objet (19) à mesurer,
b) ainsi qu'avec au moins deux bras de référence (11, 12) avec des longueurs de chemin optique fondamentales différentes l'une de l'autre correspondant à au moins une grandeur géométrique à mesurer,
c) dans lequel des moyens respectifs pour générer les rayons des bras de référence (33a, 35a) sont prévus dans les deux bras de référence (11, 12) et
**caractérisé par**
d) un seul élément de variation de la longueur du chemin (23 ; 103; 147) tournant autour d'un axe de l'élément (21, 105) agissant dans les deux bras de référence (11, 12),
e) dans lequel les deux rayons des bras de référence au moins des deux bras de référence (11, 12) au moins sont acheminés avec un angle de décalage mutuel (δ_{w}, δₛ, δₓ) sur l'élément de variation du chemin (23 ; 103 ; 147), de telle sorte que la variation de la longueur du chemin agisse d'abord dans le premier bras de référence et ensuite dans le deuxième bras de référence, du fait qu'une variation de la longueur du chemin peut s'appliquer successivement aux longueurs des chemins optiques fondamentaux.

7. Dispositif selon la revendication 6, **caractérisé en ce que** l'élément de variation du chemin (23 ; 103 ; 147) comporte plusieurs surfaces de revêtement extérieur (49a-d ; 109a-1091), l'axe de l'élément (21 ; 105) de l'élément de variation du chemin (23; 103 ; 147) étant disposé parallèlement aux surfaces de revêtement extérieur (49a-d ; 109a-1091) et perpendiculairement aux axes des rayons libres des bras de référence (33a, 35a ; 111a, 121).

8. Dispositif selon la revendication 6 ou 7, **caractérisé en ce que** l'élément de variation de la longueur du chemin (23) est en forme de polygone régulier en coupe transversale, dans lequel les rayons de référence (33b, 33c, 33d; 35b, 35c, 35d) se réfléchissent à l'intérieur de l'élément (23) sur au moins deux des surfaces du revêtement extérieur (49a-d) du polygone, et de préférence les surfaces du revêtement extérieur (49a-d) comportent au moins une surface partielle (50a-d) qui est réfléchissante à près de 100% pour les rayons des bras de référence (33b, 33c, 33d ; 35b, 35c, 35d), et chaque surface partielle (50a-d) commençant à chaque angle des surfaces du revêtement extérieur (47a-d) n'est recouverte d'un revêtement réfléchissant à près de 100% que pour la section du rayon (33b, 33c ; 35b, 35c) pouvant se réfléchir à l'intérieur de l'élément (23), qui forme avec la section du rayon (33c, 33d ; 35c, 35d) incident associé, un angle aigu (β), pour obtenir un guidage du rayon minimisant au maximum les pertes.

9. Dispositif selon une des revendications 6 à 8, **caractérisé par** une unité de focalisation (58) agissant sur le rayon libre du bras de mesure (22), disposée dans le bras de mesure (13) de l'interféromètre de Michelson, dont le lieu de focalisation peut être focalisé en alternance sur au moins deux lieux de mesure différents, dans lequel la focalisation sur les lieux de mesure correspondants peut être entreprise de manière synchronisée avec la rotation de l'élément de variation du chemin (23), et l'unité de focalisation (58) possède de préférence une unité de lentilles (59), dans lequel une unité optique (61, 63 ; 130) mobile à travers la section transversale du rayon de mesure est disposée devant ou derrière l'unité de lentilles (59), dans le chemin des rayons de mesure, dans un domaine longitudinal du rayon divergeant pour l'essentiel d'une configuration de rayons parallèles, permettant de régler diverses configurations de rayons du rayon de mesure (22) pour obtenir un déplacement du point de focalisation synchronisée avec la rotation de l'élément de variation du chemin (23), et notamment l'unité optique (61, 63 ; 130) comportant au moins un domaine plan parallèle, transparent, pouvant s'insérer dans le rayon de mesure (22) avec une longueur optique du rayon de mesure (22) définie, qui avec l'unité de focalisation (59) engendre un déplacement du point de focalisation d'une surface limite d'épaisseur à l'autre, ce domaine au moins étant disposé de telle sorte qu'il se trouve dans le rayon de mesure (22) pendant une des durées de mesure actives alternantes du bras de référence (11, 12) respectif, et l'unité optique étant de préférence réalisée sous la forme d'un disque rotatif circulaire (63 ; 130) avec au moins un segment du corps solide transparent (64 ; 135, 137).

10. Dispositif selon une des revendications 6 à 9, **caractérisé par** plusieurs sources de rayonnement d'éclairage (70), disposées devant l'objet (19), notamment devant un oeil humain comme objet (19), et avec une unité de prise de vues (73) permettant de reproduire les lieux de réflexion des sources de rayonnement d'éclairage (80a, 80b ; 89a, 89b ; 97n,m) sur la surface de l'objet (69), et une unité de traitement (74), permettant de poser les distances mutuelles des lieux de réflexion représentés par rapport aux distances des sources de rayonnement d'éclairage (80a, 80b ; 89a, 89b ; 97n,m) pour la détermination d'une cambrure superficielle de la surface de l'objet (69) sous la forme d'une autre grandeur géométrique, et des éléments de focalisation (99) disposés de préférence devant les sources de rayonnement (97n,m) permettant de focaliser le rayonnement des sources de rayonnement d'éclairage (97n,m) sur différents domaines dans l'objet transparent (19), auquel cas lorsque l'objet (19) est un oeil humain, les éléments de focalisation permettent d'entreprendre une focalisation sur la face avant de la cornée et/ou sur la face arrière de la cornée et/ou sur la face avant du cristallin et/ou sur la face arrière du cristallin, auquel cas notamment les sources de rayonnement d'éclairage (80a, 80b ; 89a, 89b ; 97n,m) sont des LEDs qui émettent de préférence un rayonnement d'une autre longueur d'onde que la source de rayonnement (1) de l'interféromètre de Michelson, et les éléments de focalisation sont de préférence des lentilles cylindriques (99a-h), auquel cas il est possible d'adapter une ou plusieurs lentilles cylindriques tournant sur les sources de rayonnement pour éviter une multitude de lentilles cylindriques.

11. Dispositif selon une des revendications 6 à 10, **caractérisé par** des guides de rayonnement (25a, 25b, 26a, 26b, 14a, 14b) permettant de guider le rayonnement des bras de référence (11, 12) et celui du bras de mesure (13) et par des éléments de couplage de sortie respectivement d'entrée disposés aux extrémités des guides de rayonnement et permettant de coupler immédiatement devant ou derrière des composants optiques (23 ;58, 70, 71) respectivement devant l'objet à mesurer (19), en sortie respectivement en entrée, le rayonnement guidé, respectivement à guider, dans le guide de rayonnement concerné sous la forme d'un rayon libre (22, 33a, 33d, 35a, 35d).

12. Dispositif selon la revendication 10, **caractérisé par** des longueurs d'ondes mutuellement distinctes de la source de rayonnement (1) à cohérence courte et au moins un premier nombre de sources de rayonnement d'éclairage (80a, 80b ; 89a, 89b ; 97n,m), par un miroir sélecteur de longueurs d'ondes (71) dans le bras de mesure (13) permettant de réfléchir le rayon (22) de la source de rayonnement (1) à cohérence courte sur l'objet (19) et permettant de transmettre le rayonnement des sources de rayonnement d'éclairage (80a, 80b ; 89a, 89b ; 97n,m), et par une unité de reproduction (72) pour le rayonnement des sources de rayonnement d'éclairage (80a, 80b ; 89a, 89b ; 97n,m) pouvant être réfléchi par l'objet (19, 69), l'axe optique du bras de mesure (13) rejoignant l'axe optique de l'unité de reproduction (72) devant l'objet (19).

13. Dispositif selon les revendications 9 et 10, **caractérisé par** un coupleur de fibre monomode (9) 3x3, dans lequel le rayonnement de mesure émergeant du coupleur de fibre monomode (9) 3x3 peut être guidé dans un premier conducteur de rayonnement (14a), et le coupleur de fibre monomode (9) 3x3 est relié à la source de rayonnement (1) de Michelson par l'intermédiaire d'un deuxième conducteur de rayonnement (3c), à deux bras de référence (11, 12) par l'intermédiaire d'un troisième et d'un quatrième conducteur de rayonnement (25a, 26a) ainsi que vers un premier et un deuxième détecteur d'interférence (15, 16) par l'intermédiaire d'un cinquième et d'un sixième conducteur de rayonnement (10a, 10b), et l'autre extrémité (14b) du premier conducteur de rayonnement (14a) est acheminée directement devant l'unité de focalisation (58), un miroir (71) sélectif de longueur d'onde est disposé après l'unité de focalisation (58) pour pouvoir diriger le rayonnement de mesure (22) sur l'objet à mesurer (19) et pouvoir guider le rayonnement de mesure (22) réfléchi par l'objet (19) en retour dans l'unité de focalisation (58) et dans le premier conducteur de rayonnement (14a, 14b), et les sources de rayonnement d'éclairage (80a, 80b ; 89a, 89b ; 97n,m) sont disposées entre le miroir (71) et l'objet (19), de préférence dans un plan perpendiculaire à l'axe du rayonnement de mesure (22) à proximité immédiate du miroir (71) et dans le prolongement vers l'arrière du rayonnement de mesure (22) sur la face du miroir (71) éloignée de l'objet (19), un système de reproduction (72) est disposé devant une unité de traitement optique (73) pour les sources de rayonnement d'éclairage (80a, 80b ; 89a, 89b ; 97n,m) réfléchies sur, respectivement dans l'objet (19).

14. Dispositif selon une des revendications 6 à 9, **caractérisé en ce qu'**à côté du tomographe de cohérence optique dans le domaine temporel (155), on dispose d'un tomographe de cohérence optique dans le domaine spectral (156), les deux tomographes (155, 156) fonctionnant avec une seule source de rayonnement (1) commune.

15. Dispositif selon la revendication 14, **caractérisé par** un spectromètre (163) et un coupleur de fibre monomode (160) 4x4, dans lequel le rayonnement de mesure pour le tomographe de cohérence optique dans le domaine temporel (155) et pour le tomographe de cohérence optique dans le domaine spectral (156) peut être guidé dans un premier conducteur de rayonnement (14a, 14b), et le coupleur de fibre monomode (160) est relié à la source de rayonnement (1) par l'intermédiaire d'un deuxième conducteur de rayonnement (3a-c), aux deux bras de référence (11, 12) qui comportent un élément commun de variation de la longueur du chemin (23) par l'intermédiaire d'un troisième et d'un quatrième conducteur de rayonnement (25a, 26a), vers un premier et un deuxième détecteur d'interférence (15, 16) par l'intermédiaire d'un cinquième et d'un sixième conducteur de rayonnement (10a, 10b), avec un troisième bras de référence (176) par l'intermédiaire d'un septième conducteur de rayonnement (177a) et avec le spectromètre (163) par l'intermédiaire d'un huitième conducteur de rayonnement (161).
